(19) Europäisches Patentamt
European Patent Office
Office européen des brevets

(11) EP 2 808 373 A1

(12) EUROPEAN PATENT APPLICATION

(43) Date of publication:
03.12.2014 Bulletin 2014/49

(51) Int Cl.:
*C09K 11/06* (2006.01) *H01L 51/00* (2006.01)

(21) Application number: 13169826.8

(22) Date of filing: 29.05.2013

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME

(71) Applicant: Solvay SA
1120 Brussels (BE)

(72) Inventors:
- Dabeux, François
  1140 Evere (BE)
- Caille, Jean-Raphael
  5000 Namur (BE)
- Fenoll, Mathieu
  1030 Schaerbeek (BE)
- Bolsee, Jean-Christphe
  1120 Brussels (BE)

(74) Representative: Dr. Langfinger & Partner
In der Halde 24
67480 Edenkoben (DE)

# (54) Semiconductor materials on the basis of rylene diimide derivatives or napththalene derivatives, and polymers and semiconductor devices comprising said materials

(57) Novel organic semiconductors based on rylene tetracarboxylic diimides or naphthalene tetracarboxylic diimides comprising a bridging group and uses thereof as organic semiconductors in organic electronic devices, in particular in Organic Field Effect Transistors (OFET).

Figure 1

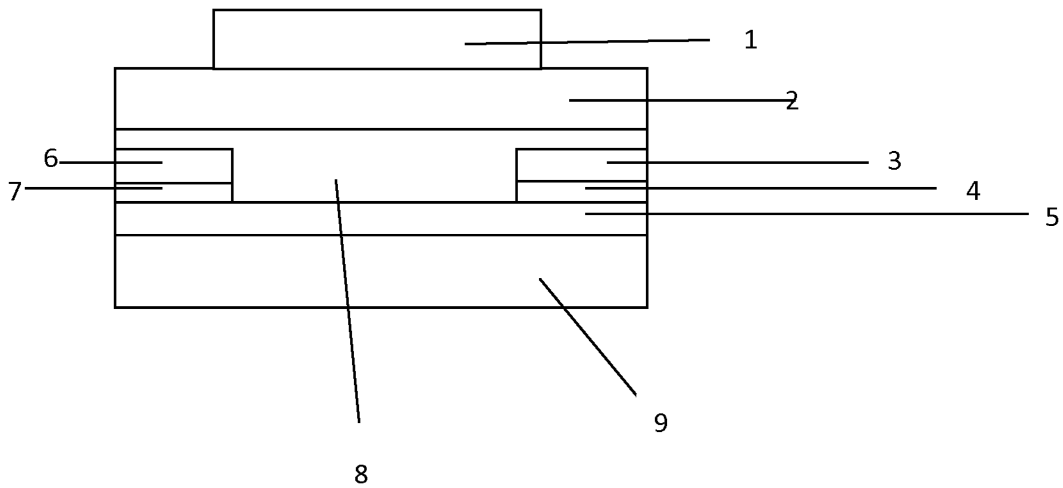


EP 2 808 373 A1

## Description

The present invention relates to novel organic semiconductors and their use in organic electronic devices.

Rylene diimide derivatives and naphthalene diimide derivatives have been intensively studied as electron transporting materials for the fabrication of p-n-junctions, photovoltaic cells, field-effect-transisotors or other electronic devices. Marks et al., Adv. Mater. 2011, 23, 268-284 provide an overview of the compounds which have been investigated in this regard.

While quite a number of organic semiconductor small molecules, oligomers and/or polymers have been described in the prior art, it has generally been a problem to provide organic semiconductor compounds which can efficiently transport electrons (especially under ambient conditions, i.e. in the presence of air and/or water).

Yan and Zhao (Org. Lett. 2009, 11 (15) 3426-3419) reported the synthesis and characterization of several solution processable bis(PDI) oligomeric compounds bridged by phenyl or acetylene groups, as shown below:

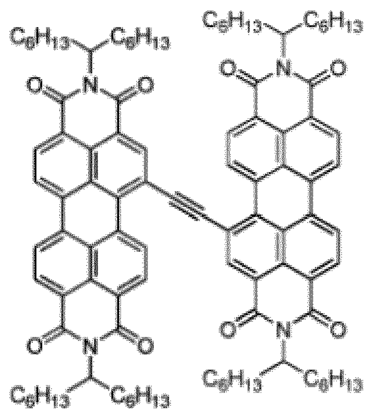

and

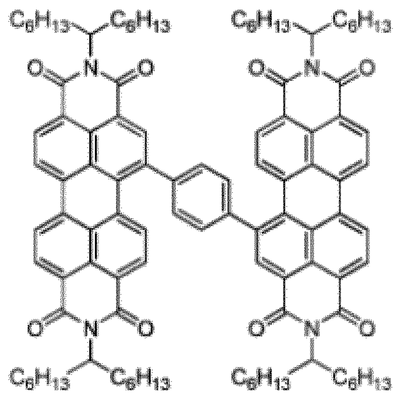

.

Naphthalene diimide (NDI) derivatives with a bridging group linking two NDI units have also been described as organic semiconductors in WO 2012/142460, represented by the following general structure

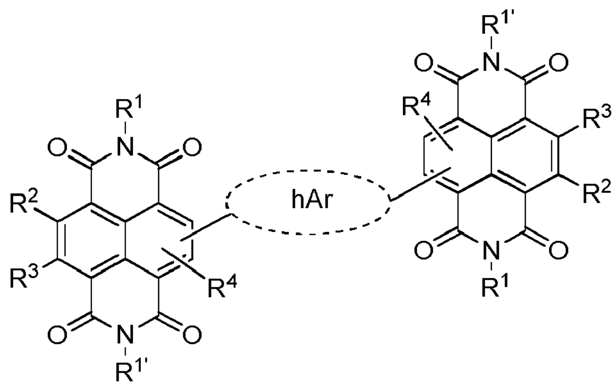

wherein hAr is represented by the following structures

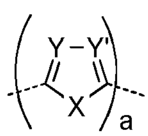

or

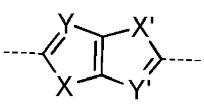

or

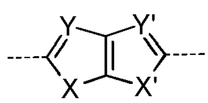

or

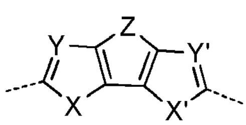

or

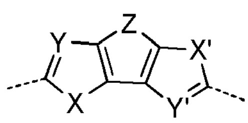

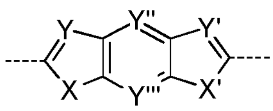

or

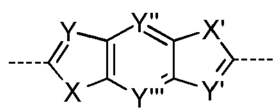

or

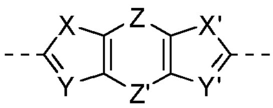

or

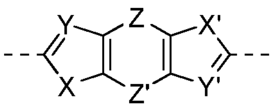

or

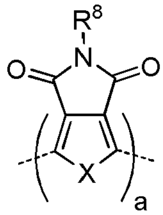

or

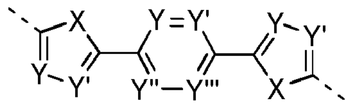

wherein

i. "a" is an integer 1, 2, 3, or 4;

ii. each X and X' is independently selected from O, S, Se, or $NR^6$, wherein $R^6$ is a $C_1$-$C_{30}$ organic group independently selected from normal, branched, or cyclic alkyl, fluoroalkyl, aryl, heteroaryl, alkyl-aryl, and alkyl-heteroaryl groups optionally substituted with one or more fluoro, cyano, alkyl, alkoxy groups;

iii. each Y, Y', Y" and Y'" is independently selected from N, and $CR^7$, where $R^7$ is hydrogen, fluoro, or a $C_1$-$C_{30}$ organic group independently selected from cyano, normal, branched, or cyclic alkyl, perfluoroalkyl, alkoxy, perfluoroalkoxy, aryl, heteroaryl, alkyl-aryl, and alkyl-heteroaryl groups, optionally substituted with one or more fluoride, cyano, alkyl, alkoxy groups;

iv. each Z and Z' is independently selected from O, S, Se, $C(R^8)_2$, $Si(R^8)_2$, $NR^8$, (CO), $(CO)_2$ or $C=C(CN)_2$, wherein $R^8$ is a $C_1$-$C_{30}$ organic group independently selected from normal, branched, or cyclic alkyl, perfluoroalkyl, aryl, heteroaryl, alkyl-aryl, and alkyl-heteroaryl groups, optionally substituted with one or more fluoro, cyano, alkyl, alkoxy groups;

b) each $R^1$ and R1' is a $C_1$-$C_{30}$ organic group independently selected from a normal, branched, or cyclic alkyl, aryl, heteroaryl, alkyl-aryl, or alkyl-heteroaryl group optionally substituted with one or more halide, cyano, alkyl, or alkoxy groups;

c) $R^2$, $R^3$, and $R^4$ are independently selected from hydrogen, halide, or a $C_1$-$C_{30}$ organic group independently selected from cyano, normal, branched, or cyclic alkyl, fluoroalkyl, aryl, heteroaryl, alkyl-aryl, acyl- and alkyl-heteroaryl groups, optionally substituted with one or more fluoro, cyano, alkyl or alkoxy groups.

Most of these compounds are ambipolar in nature, only few of them being pure electron transporting materials. Albeit these materials provide an interesting property spectrum, improvement in certain regards would be desirable.

One of the existing needs is to provide semiconductors with reduced or suppressed ambipolarity which may be used as n-type semiconductors in organic electronic devices.

Furthermore, to be used in solvent processing techniques, which is generally preferred, the solubility of the semiconductors has to be sufficiently high.

Accordingly, there was a need for further organic semiconductors which can be used in organic electronic devices having a balanced property spectrum as outlined above.

It was thus an object of the present invention to provide additional organic semiconductors based on rylene diimides and in particular naphthalene diimides.

This object has been achieved with the novel compounds in accordance with claim 1 and the polymers in accordance with claim 10.

Preferred embodiments of the present invention are set forth in the dependent claims and the detailed description hereinafter.

The present invention relates to compounds of general formulae (1) or (2)

$$XDI\text{-}Y\text{-}XDI \qquad (1)$$

or

$$Y'\text{-}XDI'\text{-}Y' \qquad (2)$$

wherein XDI, which may be the same or different at each occurrence, represents a monovalent rylene tetracarboxylic acid diimide and in particular a monovalent naphthalene tetracarboxylic acid diimide substituent, bound to Y via one or two aromatic rings wherein one or more of the oxygen atoms of the imide groups may be replaced by sulfur atoms, XDI', which may be the same or different at each occurrence, represents a divalent rylene tetracarboxylic acid diimide substituent and in particular a divalent naphthalene tetracarboxylic acid diimide substituent, bound to Y' via one or two aromatic rings, wherein one or more of the oxygen atoms of the imide groups may be replaced by sulfur atoms Y represents a divalent substituent selected from the group consisting of formulae (3) to (6)

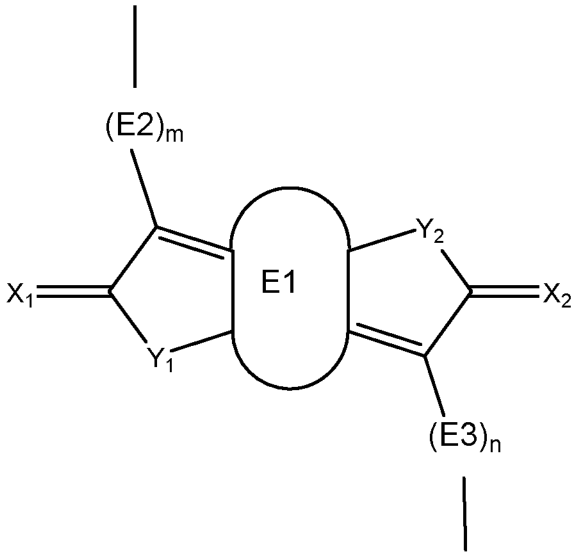

(3)

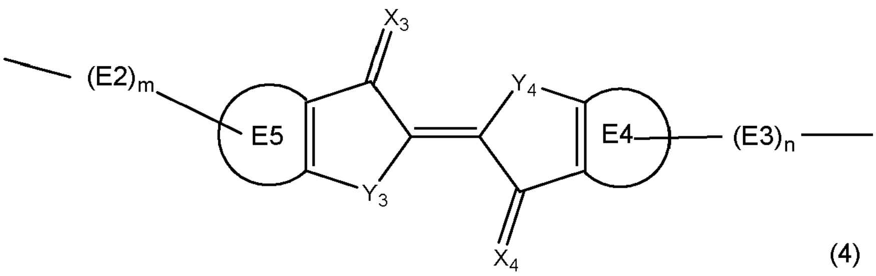

(4)

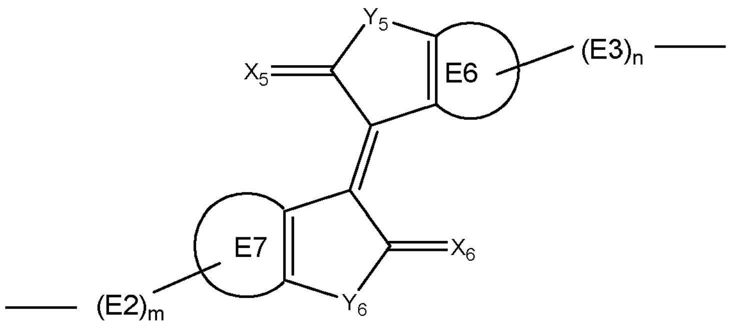

(5)

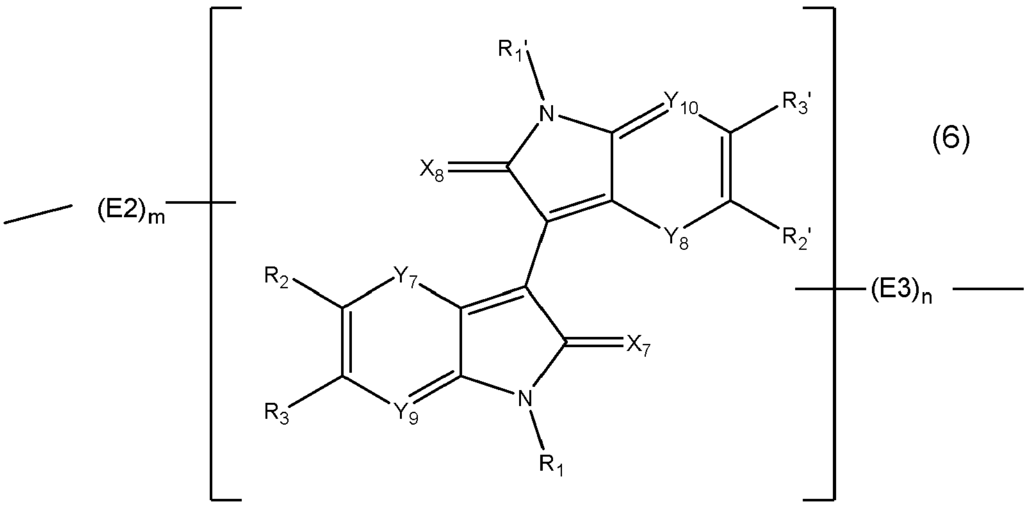

(6)

Y' represents a monovalent substituent represented by formulae (3') to (6')

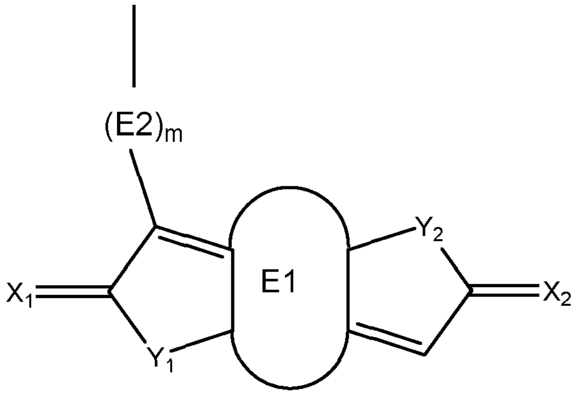

(3')

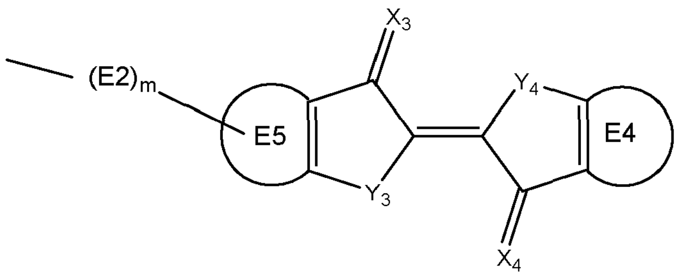

(4')

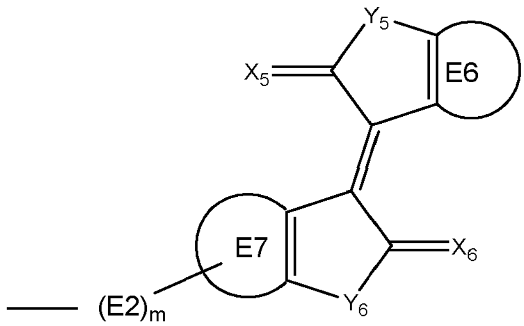

(5')

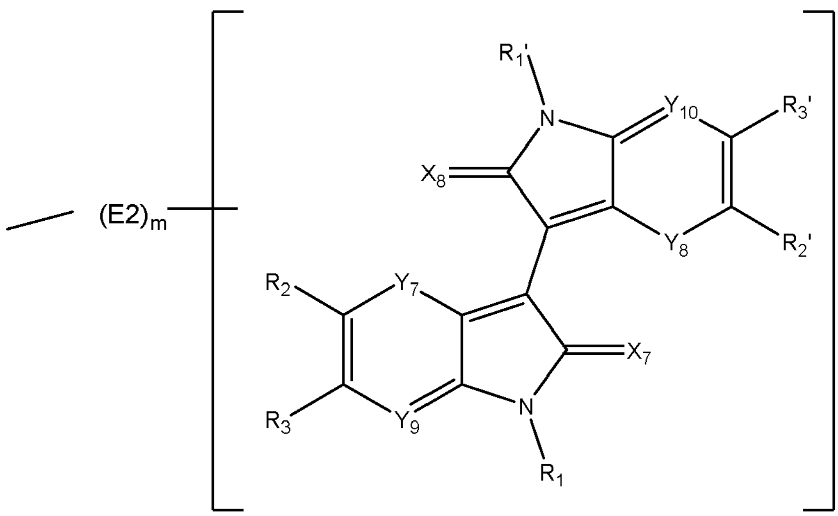

(6')

wherein $X_1$, $X_2$, $X_3$, $X_4$, $X_5$, $X_6$, $X_7$ and $X_8$ which may be the same or different at each occurrence, are selected from O, S or NR,

$Y_1$, $Y_2$, $Y_3$, $Y_4$, $Y_5$, $Y_6$, $Y_7$ and $Y_8$, , which may be the same or different, are selected from O, S or NR,

$Y_9$ and $Y_{10}$, which may be the same or different, are N or P,

R, $R_1$ and $R_1$', which may be the same or different, are selected from $C_1$-$C_{20}$ hydrocarbyl or $C_1$-$C_{20}$ heterohydrocarbyl groups

$R_2$, $R_2$', $R_3$ and $R_3$', which may be the same or different at each occurrence, represent hydrogen, halogen or a $C_1$-$C_{20}$ hydrocarbyl or $C_1$-$C_{20}$ heterohydrocarbyl group or $R_2$ and $R_3$ respectively $R_2$' and $R_3$' form together a substituted or unsubstituted five or six membered carbocyclic or heterocyclic, aromatic or heteroaromatic ring, which ring may be part of a fused ring system,

E1 represents a carbocyclylene, heterocarbocyclylene, arylene or heteroarylene group which is fused through two vicinal carbon atoms with each of the two five membered rings shown in formula (3),

E2 and E3 represent a carbocyclylene, heterocarbocyclylene, arylene or heteroarylene ring system comprising 4 to 14 carbon atoms, n and m, independent of one another, are 0 or 1,

E4, E5, E6 and E7, which may be the same or different at each occurrence, represent a carbocyclenyl, heterocarbocyclenyl, aryl or heteroaryl ring system comprising 4 to 14 carbon atoms which is fused to the 5- membered ring system shown in formula 4 and 5 through two vicinal carbon atoms.

In accordance with a first preferred embodiment of the present invention $X_1$ to $X_8$ represent an oxygen atom.

According to another preferred embodiment of the present invention $Y_1$ to $Y_8$ represent oxygen or an NR group, especially preferred an NR group.

$Y_9$ and $Y_{10}$ may be the same or different and are N or P, preferably $Y_9$ and $Y_{10}$ are N.

R, $R_1$ and $R_1$', which may be the same or different at each occurrence, are selected from $C_1$-$C_{20}$ hydrocarbyl or $C_1$-$C_{20}$ heterohydrocarbyl groups, which may be linear, branched or cyclic. Preferred groups R, $R_1$ and $R_1$' are linear or branched alkyl groups having 1 to 20 carbon atoms, preferably 2 to 18 and even more preferably 4 to 18 carbon atoms. By way of example ethyl, butyl, pentyl, hexyl, octyl or decyl groups may be mentioned here.

Preferred substituents R, $R_1$ and $R_1$' are

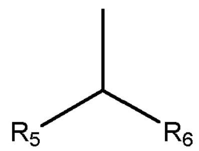

and

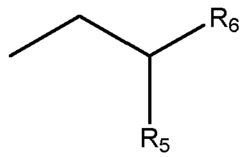

wherein $R_5$ and $R_6$, which may be the same or different at each occurrence, are $C_2$-$C_{12}$ alkyl groups, preferably $C_4$ to $C_{12}$ alkyl groups, particularly preferred $C_4$ to $C_{10}$ alkyl groups, e.g. ethyl, butyl, pentyl, hexyl, octyl or decyl groups, which might be linear or branched, preferably linear.

Preferably $R_2$ and $R_3$ respectively $R_2$' and $R_3$' form together a five or six membered aromatic or heteroaromatic ring, which may be part of a fused ring system and especially preferably $R_2$ and $R_3$ respectively $R_2$' and $R_3$' form an aromatic ring. In any case, the ring system formed by $R_2$ and $R_3$ respectively $R_2$' and $R_3$' may carry substituents.

E1 in formulae (3) respectively (3') represents carbocyclylene, heterocarbocyclylene, arylene or heteroarylene ring system. E1 is fused to each of the two five membered rings; thus a total of four ring atoms of E1 are involved in the binding of these two five membered rings.

The bonding to the two five membered rings systems is achieved through carbon atoms so that ring systems E1 comprise at least four carbon atoms.

Preferred examples for E1 are arylene or heteroarylene with arylene groups being particularly preferred. Especially preferred arylene groups are phenylene, naphthylene or anthracenylene.

Accordingly, especially preferred groups of formulae (3) and (3') are derived from the following E1 groups

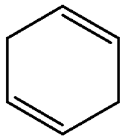

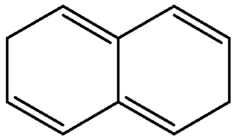

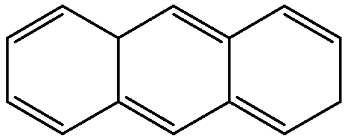

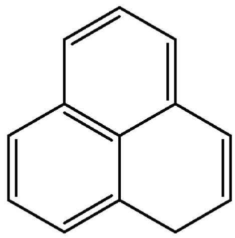

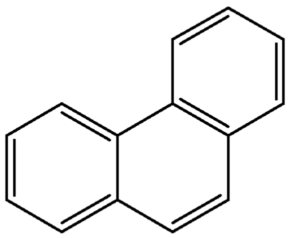

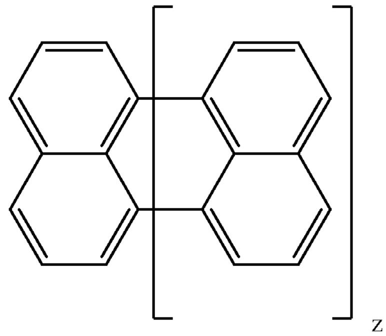

wherein z represents an integer of from 1 to 3, with phenylene and naphthylene cores being particularly preferred.

The bonding of E1 to the two five membered rings may be through any two vicinal carbon atoms at the periphery of E1.

E2 and E3 preferably represent a $C_4$ to $C_{14}$ arylene or heteroarylene group, which may be substituted or unsubstituted with halogen, a $C_1$-$C_{20}$ hydrocarbyl or a $C_1$-$C_{20}$ heterohydrocarbyl group, with arylene groups being particularly preferred. Most preferred are substituted or unsubstituted phenylene groups.

E4 to E7, independent of one another, preferably are represented by formula 7

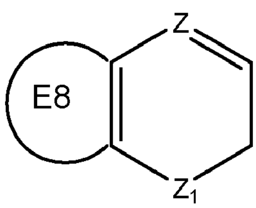

(7)

wherein

E8 represents a substituted or unsubstituted five or six membered aromatic or heteroaromatic ring,

Z represents N or P and

$Z_1$ represents O, S, NR'' or PR''' where R'' and R''' may be the same or different at each occurrence and represent hydrogen or a $C_1$-$C_{20}$ hydrocarbyl or $C_1$-$C_{20}$ heterohydrocarbyl group and wherein the XDI substituent is attached to the aromatic or heteroaromatic ring E8 in formula (7) and the heterocyclyl ring in formula (7) is fused to the five membered ring in formulae (4), (4'), (5) and (5') and wherein both rings may be substituted or unsubstituted.

Particularly preferred Z is nitrogen and/or $Z_1$ is a group -NR'' as defined above.

Preferred examples for R'' and R''' are alkyl, alkenyl, carbocyclyl, heterocarbocyclyl, aryl or heteroaryl groups, all with 1 to 20 carbon atoms, which may be substituted or unsubstituted. The alkyl and alkenyl groups may be linear or branched.

The five or six membered aromatic or heteroaromatic ring is preferably selected from the following ring systems

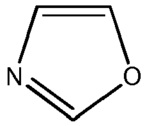 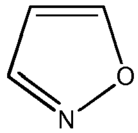 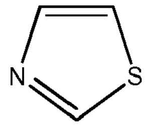 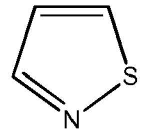 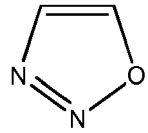

oxazole isoxazole thiazole isothiazole 1,2,3-oxadiazole

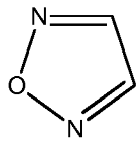 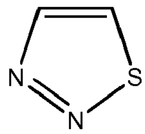 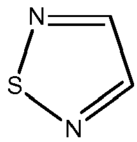 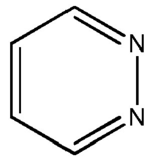

1,2,5-oxadiazole 1,2,3-thiadiazole 1,2,5-thiadiazole pyridazine

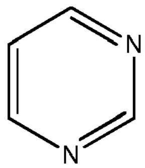 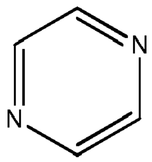 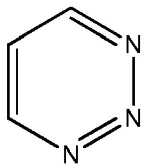 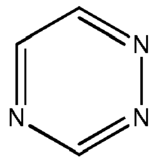 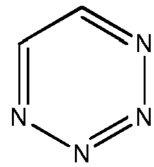

pyrimidine pyrazine 1,2,3-triazine 1,2,4-triazine 1,2,3,4-tetrazine

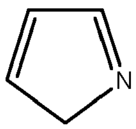 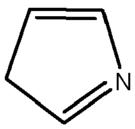 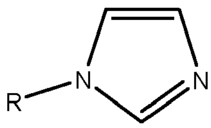 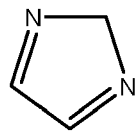 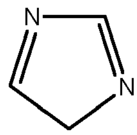

2 *H*-pyrrole 3 *H*-pyrrole 1-substituted 1 *H*-imidazole 2H-imidazole 4 *H*-imidazole

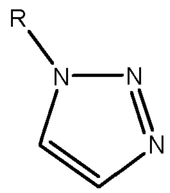 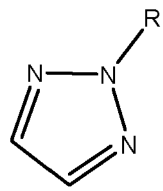 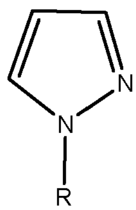

1-substituted-1 *H*-1,2,3-triazole 2-substituted-2H-1,2,3-triazole 1-substituted-1 *H*-pyrazole

wherein R represents a substituent.

Particularly preferred as E8 are substituted or unsubstituted phenylene rings.

The units Y and Y' represented by formulae (4) and (4') are based on indigo derivatives whereas units Y and Y' represented by formulae (5) and (5') are based on isoindigo derivatives.

Units Y and Y' in accordance with formulae (6) and (6') are based on a leuco isoindigo core which may also be referred to as dihydrogeno isoindigo core.

In accordance with another preferred embodiment of the present invention, XDI respectively XDI' are based on a core of formula (8)

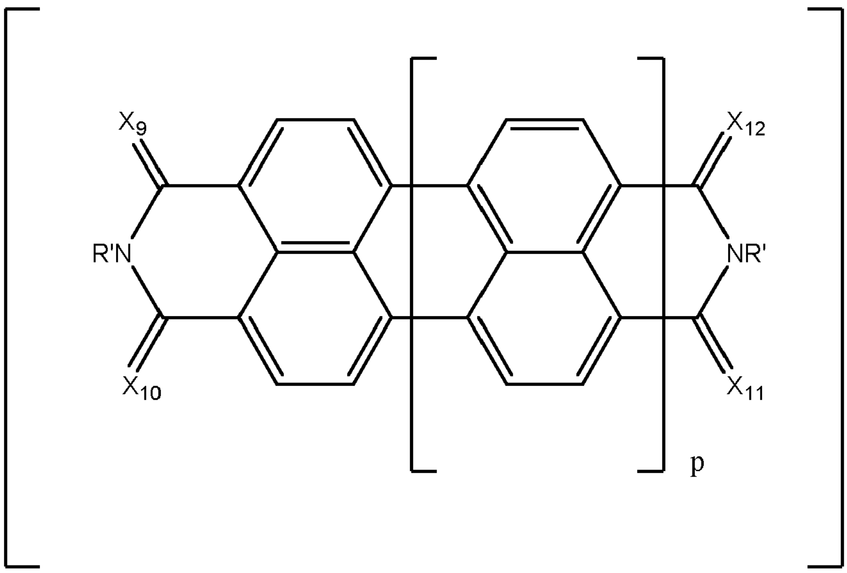

(8)

wherein $X_9$ to $X_{12}$, which may be the same or different, are S or O, R and R' are as defined in claim 1 and p represents an integer of from 0 to 3, preferably 0 or 1. and wherein all aromatic rings may be substituted or unsubstituted and where the bond to Y respectively the bond to Y' are through any of the aromatic rings.

Such compounds are generally referred to as rylene diimides and have been described in a review by Zhan et al. in Adv. Mater. 2011, 268-284 to which reference is made here.

Rylene diimides for the purpose of the present invention share the common feature of two six membered dicarboxylic acid imide rings fused to terminal naphthalene units. The three simplest rylene diimide systems are naphthalene-1,8:4,5 tetracarboxylic acid diimides (hereinafter referred to as NDIs), perylene-3,4:9,10-tetracarboxylic acid diimides (hereinafter referred to as PDIs and terylene-3,4:11.12-tetracarboxylic acid diimides reproduced below

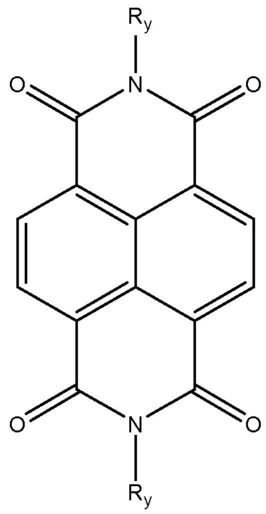

NDI

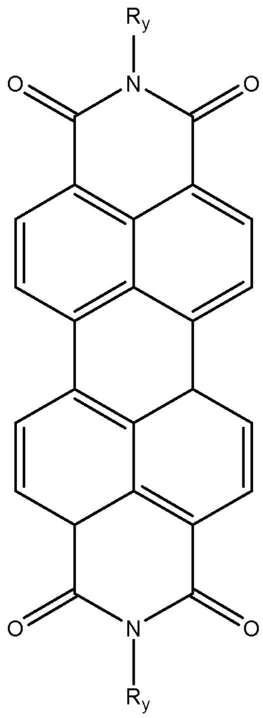

PDI

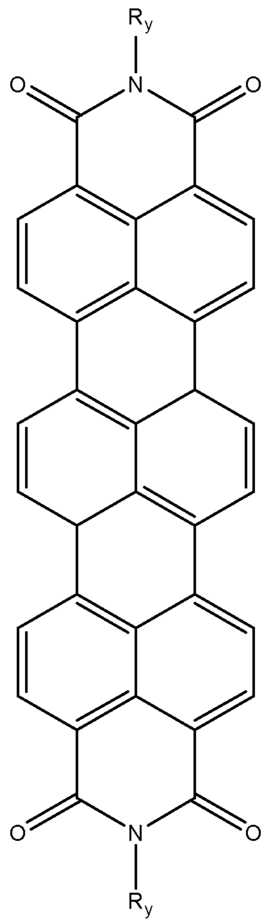

Terylene diimide

where the aromatic rings may be substituted or unsubstituted and where one or more of the oxygen atoms may be replaced by sulfur.

Suitable rylene diimides are known and commercially available or have been described in the literature or may be obtained by processes known to the skilled person so that no further details are necessary here.

Naphthalene diimide derivatives and perylene diimide derivatives are generally preferred.

In accordance with still another preferred embodiment of the present invention $Y_9$, $Y_{10}$ and Z are N.

Particularly preferred compounds with Y derived from formula (3) are the following

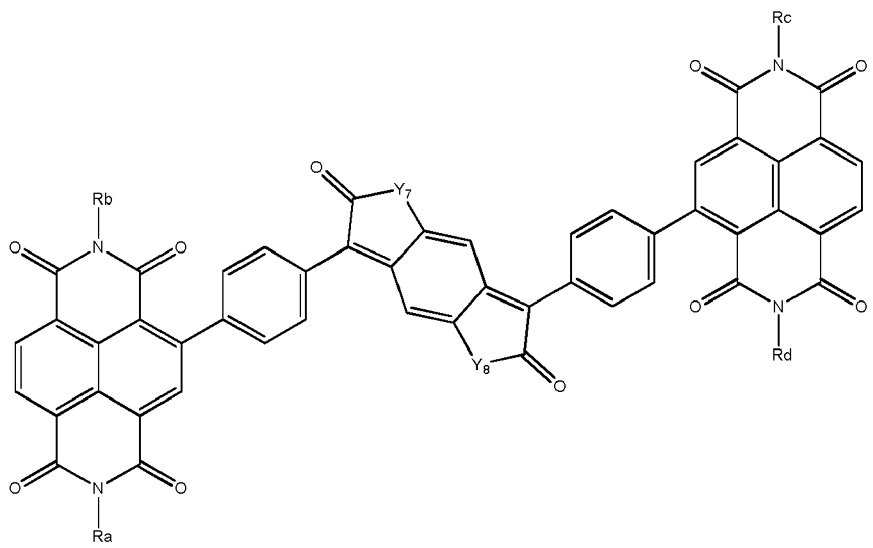

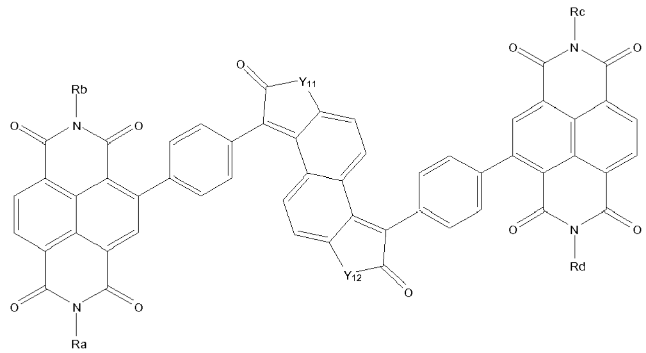

wherein $R_a$ to $R_d$, which may be the same or different, are a $C_1$ to $C_{20}$ linear or branched alkyl group and $Y_{11}$ and $Y_{12}$ represent O, S or $NR_e$ wherein $R_e$ has the meanings as defined for $R_a$ to $R_d$,

whereas particularly preferred compounds with Y or Y' derived from formula (4) respectively formula (5) are the following

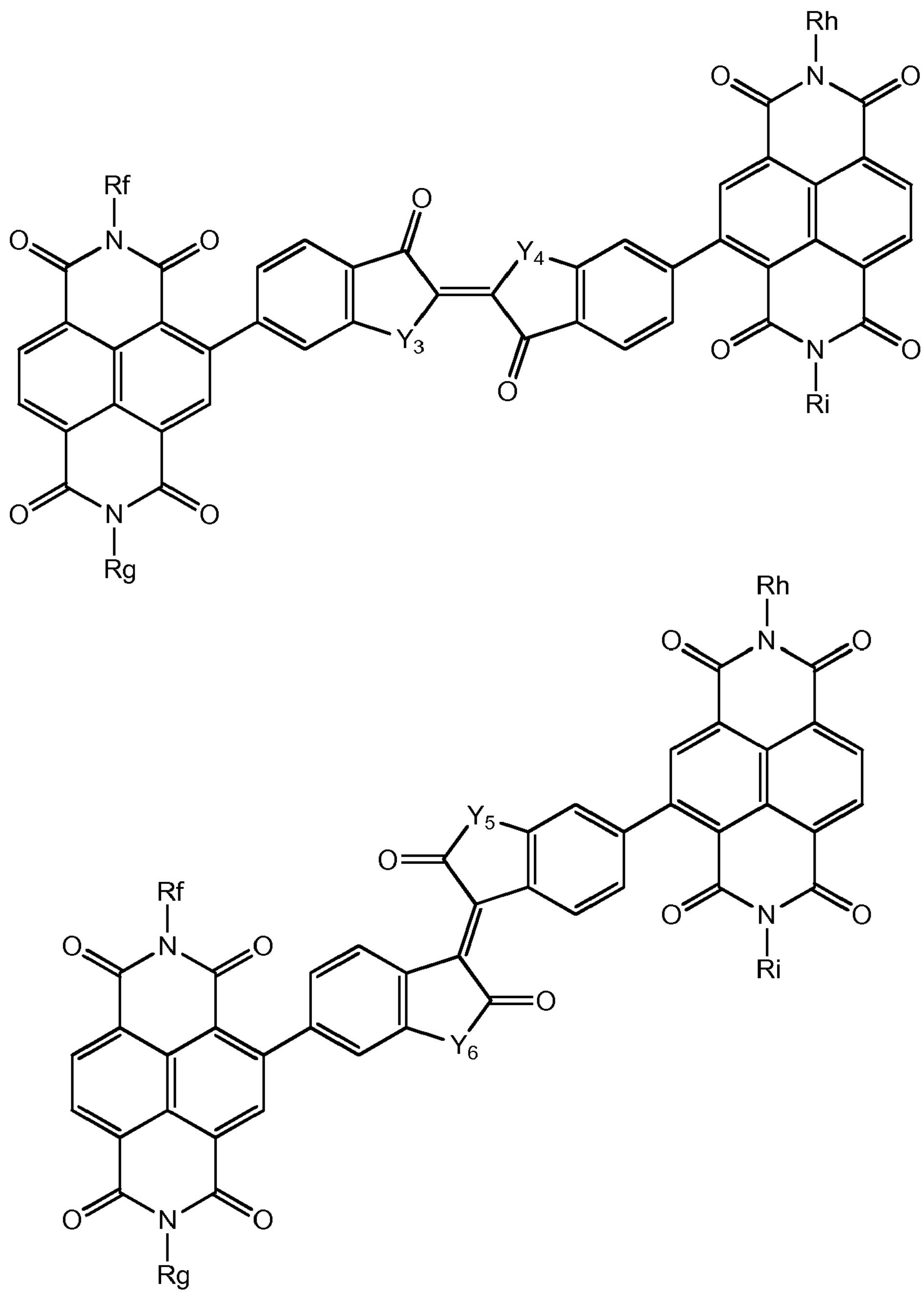

wherein $Y_3$ to $Y_6$, which may be the same or different at each occurrence, are as defined in claim 1 and $R_f$ to $R_i$, which may be the same or different at each occurrence, represent a $C_1$ to $C_{20}$ linear or branched alkyl group.

Preferred cores for groups Y respectively Y' of formula (6) respectively (6') are the following

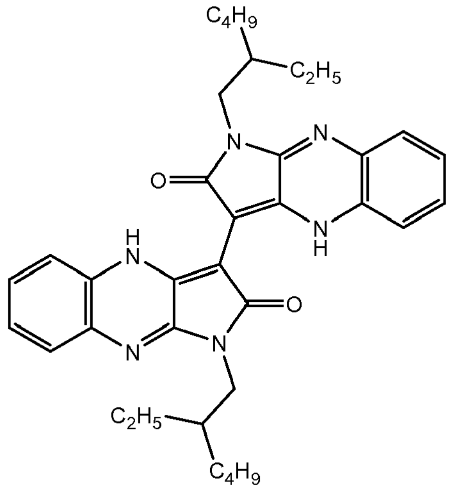

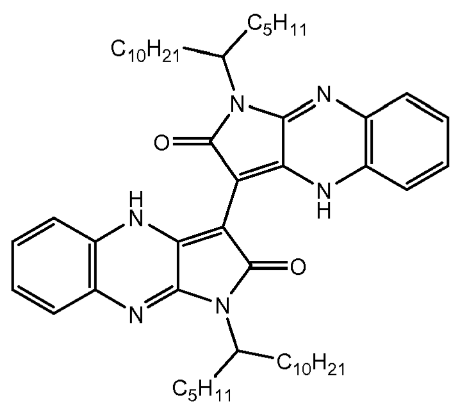

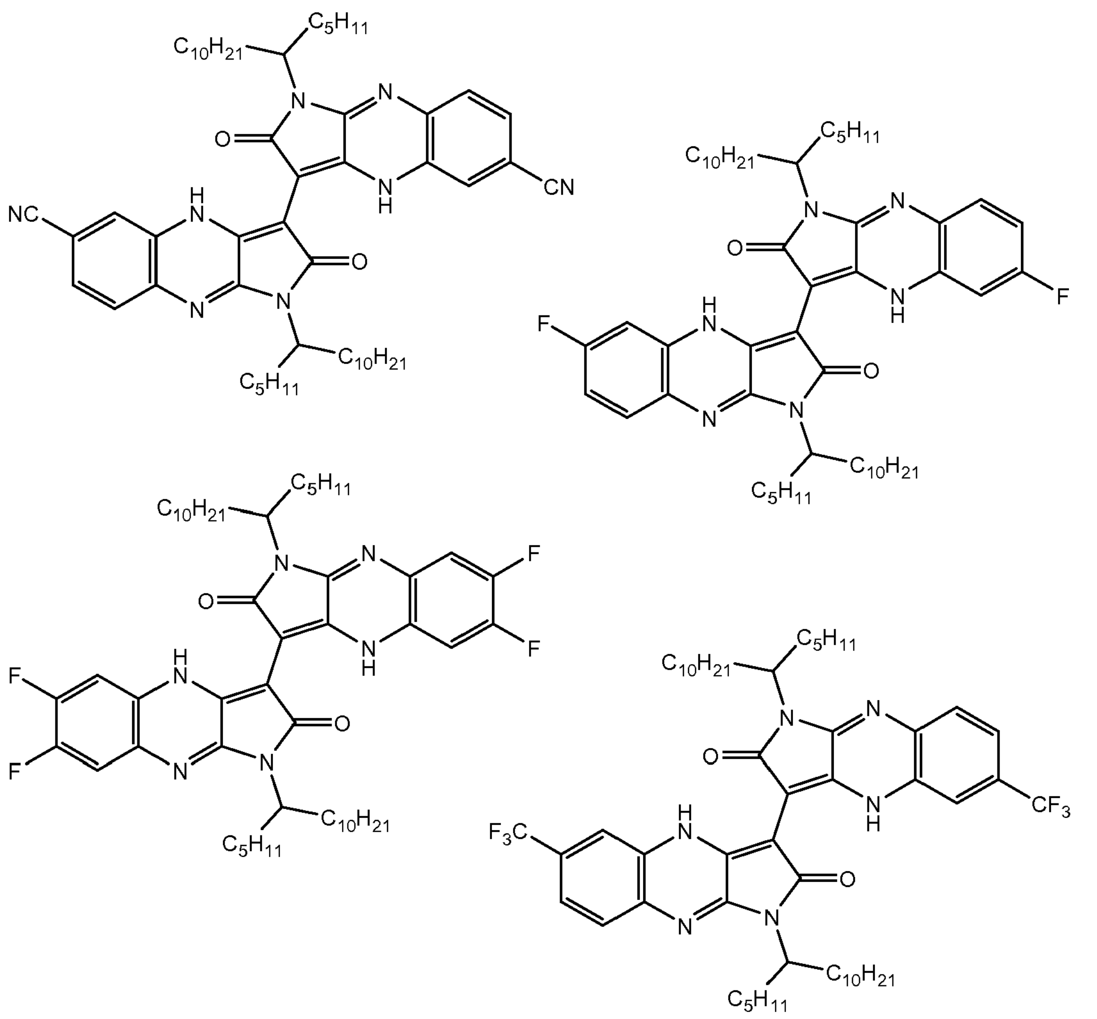

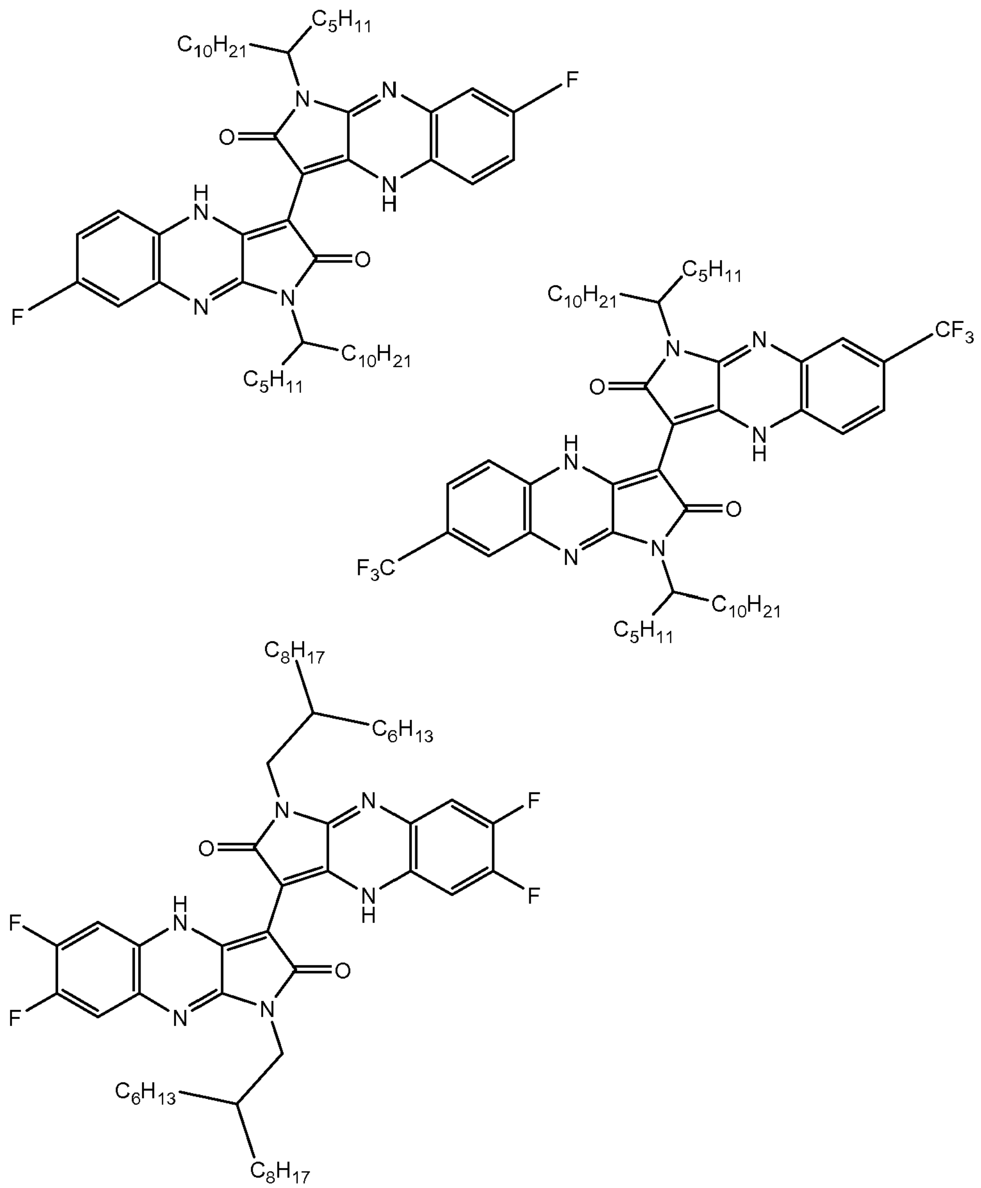

Certain of the core compounds for groups Y and Y' of formula (6) respectively (6') can occur in a number of tautomeric forms and for the purpose of the present invention, all these tautomers are included and encompassed.

The compounds of formula (2) differ from the compounds of formula (1) in that XDI' is a divalent group having a core as defined and explained above for monovalent group XDI and Y' is a monovalent group based on the core of bivalent group Y as defined above. The term monovalent, as used herein, depicts a group providing one covalent bonding site whereas the term divalent depicts a group providing two covalent bonding sites.

Preferred compounds of formula (2) are derived from the preferred cores as defined in more detail above for XDI and Y, to which reference is made at this point.

The compounds in accordance with the present invention may be obtained preferably by a coupling reaction like a Stille-, Suzuki-, Heck- or Buchwald-Hartwig reaction in accordance with the general reaction schemes

$$2\ XDI\text{-}A + B\text{-}Y\text{-}B \rightarrow XDI\text{-}Y\text{-}XDI + 2\ AB$$

$$2\ Y'\text{-}A' + B'\text{-}XDI'\text{-}B' \rightarrow Y'\text{-}XDI'\text{-}Y' + 2\ A'B'$$

wherein A and B respectively A' and B' are leaving groups in such coupling reactions.

Suitable leaving groups as well as the reaction conditions and the reactants of these coupling reactions are known to the skilled person and have been described in the literature so that further details need not be given here. The skilled person will chose the respective leaving group and the reaction conditions based on the professional knowledge

and the circumstances of the specific case to achieve the desired result.

The core compounds for units Y respectively Y' of formula (6) respectively (6'), which are novel compounds, may be obtained as exemplified in the following reaction scheme for compounds with specific meanings for certain substituents. The skilled person, based on his professional knowledge, will modify the reaction conditions and the reagents to obtain compounds of formulae (1) and (2) with other combinations of substituents.

Step 1

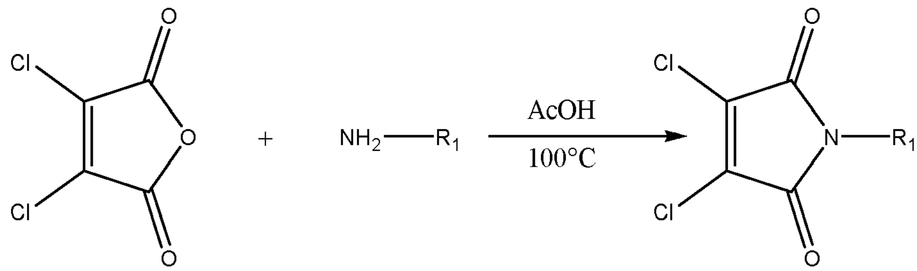


wherein Rf is preferably an optionally substituted alkyl chain

Step 2

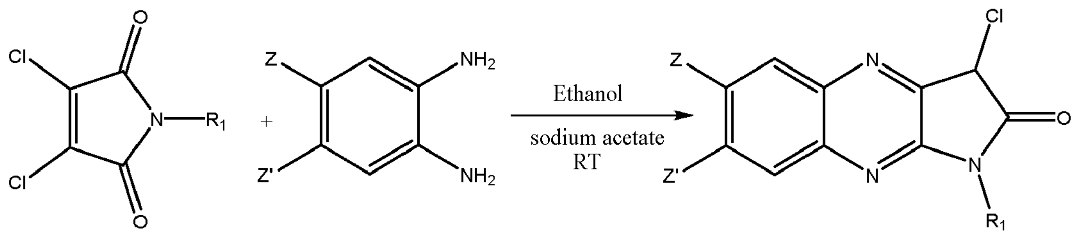


Step 3

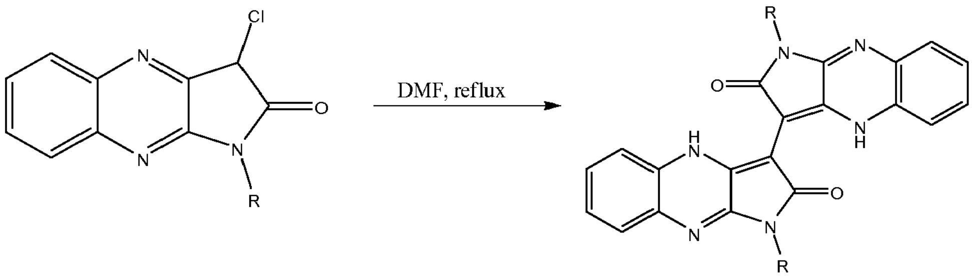


It is readily apparent to the skilled person how to modify the reaction conditions, the solvents and the starting materials to obtain specific desired compounds of formulae so that further detailed information is not necessary here.

Suitable preferred diamines which may be used in step 2 of the reaction scheme depicted above may be represented by the general formula

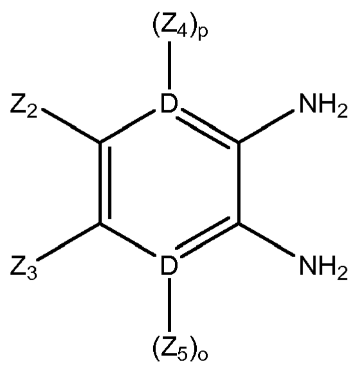

wherein

$Z_2$ to $Z_5$, independent of one another, may be hydrogen, halogen, -CN, - COOH, -COOR$_z$, C(=O)-R$_z$, OH, OR$_z$, $C_1$-$C_8$ alkyl, $C_1$-$C_8$ haloalkyl, -SCN, - $NH_2$, -N(R$_z$R$_{z'}$), -(O-$CH_2$-$CH_2$)$_n$-OH, substituted or unsubstituted $C_5$-$C_{30}$ aryl, substituted $C_2$-$C_{20}$ heteroaryl, $C_2$-$C_{20}$ alkenyl, $C_2$-$C_{20}$ alkinyl, Sn(R$_z$)$_3$, B(OR$_z$)$_2$ and -Si(R$_z$)$_3$, D and D', which may be the same or different, may be C, N or P, preferably C or N, and o and p may be 0 or 1.

Diamines wherein one of $Z_2$ and $Z_3$ represents Sn(R$_z$)$_3$, halogen or boronic acid esters are preferred if the compounds obtained from such diamines are used as reagents for the synthesis of oligomers or homo- or copolymers.

$R_z$ and $R_{z'}$, which may be the same or different, are selected from $C_1$-$C_{20}$ hydrocarbyl groups or $C_1$-$C_{20}$ heterohydrocarbyl groups, preferably from $C_1$-$C_{12}$ alkyl groups.

Preferred substituents $Z_4$ and $Z_5$ are hydrogen, halogen or $C_1$-$C_{12}$ alkyl groups.

As is immediately apparent to the skilled person, compounds in which D and D' are C and $Z_4$ and $Z_5$ are H are derivatives of 1,2-diaminobenzene. Replacing one C by N yields derivatives of 2,3-diaminopyridine whereas compounds with both D and D' being nitrogen yield derivatives of 2,3-diamino-1,4-pyrazine.

$Z_2$ and $Z_4$ respectively $Z_3$ and $Z_5$ may also form together a ring.

Just by way of example a number of suitable diamines of the above general formula are given below

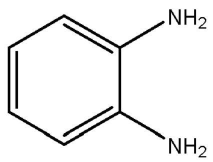
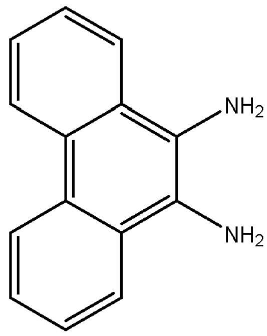
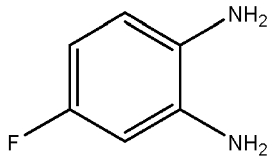
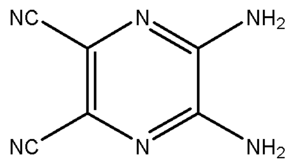
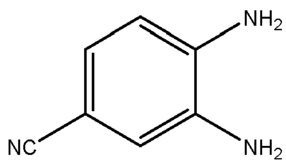

Further suitable diamines, just given by way of example, are the following, which comprise a five membered heteroaryl ring instead of a six membered ring to which the amino groups are attached

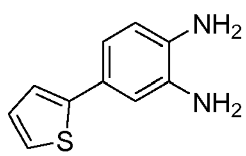
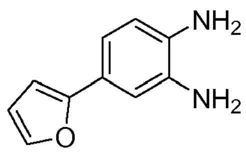
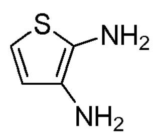
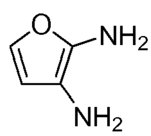
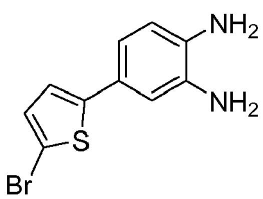
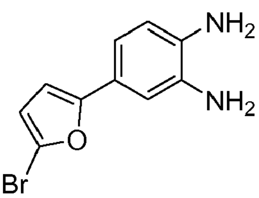
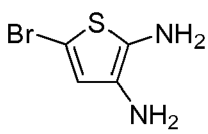
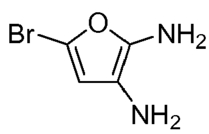

Phenanthrene-9,10-diamine and diaminophenazine and derivatives may also be mentioned here as suitable diamines.

Another embodiment of the present invention are polymers comprising repeating units derived from compounds

of formula (1) or (2) which contain at least two respective units.

Such polymers may be obtained by methods known to the skilled person and described in the literature so that no further details need to be given here.

For use in organic electronic devices the compounds in accordance with the present invention may be formed into semiconducting films by a variety of methods known to the skilled person and described in the literature. Vapor deposition methods as well as solution-based methods may be mentioned here. Such semiconducting films constitute another embodiment of the present invention.

Preferably the semi-conducting films are obtained in the form of self-assembled monolayers (SAM), i.e. molecular assemblies formed spontaneously on surfaces by adsorption or chemisorption of head groups onto a substrate from either the vapor or liquid phase.

By selecting the suitable type of head group the properties of such self assembled monolayers can be adjusted in accordance with the needs of the specific application.

The compounds in accordance with the present invention, represented by formulae (1) and (2), may be used for forming one or more layers of organic electronic devices or may constitute a component of such layers in organic electronic devices.

The compounds in accordance with the present invention or the oligomers, homo-or copolymers in accordance with the present invention may be ambipolar in nature or may show electron conductivities and hole conductivities differing by several orders of magnitude, which makes them particularly suitable for use in electron transport layers or hole transport layers of organic electronic devices.

Especially for the use in organic field effect transistors (OFET) such difference in the conductivity for different charge carriers can be advantageous.

Three essential components of field-effect transistors are the source, the drain and the gate. Field-effect transistors usually operate as a capacitor and are frequently composed of two plates. One plate works as a conducting channel between two ohmic contacts which are called the source and the drain. The other plate works to control the charge induced into the channel and is called the gate. The direction of the movement of the carriers in the channel is from the source to the drain.

Field effect transistors are unipolar transistors as they rely on single carrier-type operation and therefore a difference in conductivity for different charge carriers may be advantageous when the compounds or compositions of the present invention are used in field effect transistors.

The compounds in accordance with the present invention may also be used as semiconducting films in organic photovoltaic devices.

The compounds in accordance with the present invention are particularly suitable as efficient organic semiconductors, either in the form of small molecules or as building blocks in polymeric architectures.

In many cases the compounds in accordance with the present invention are efficient n-type organic semiconductors showing a good charge transport and a good stability under ambient conditions.

By appropriate substitution of the conjugated cores with electron withdrawing groups the HOMO and LUMO of the compounds can be tuned in accordance with the specific needs of the individual application. By grafting various chains the solubility and the pi-stacking can be promoted and the organic semiconducting properties are generally improved.

The mobility of the compounds in accordance with the present invention is improved compared to compounds known from the prior art, which can also be seen from the following examples.

Due to the good solubility of the compounds in accordance with the present invention, thin films can be prepared by e.g. spin coating out of different solvents with good reproducibility.

High performance organic semiconductors can advantageously be used for flexible electronic applications like e.g. display backplanes or RFID tags. Another example are so called E-readers.

The mobility required for such devices has to reach certain minimum values and the compounds or the compositions in accordance with the present invention show good mobilities and thus can form the basis for the development of respective organic electronic devices.

The compounds in accordance with the present invention are in particular in many cases useful as n-type semiconductors in organic field effect transistors as the electron conductivity in many cases is significantly higher than the p-type conductivity.

Accordingly, another embodiment of the present invention is related to organic electronic devices, in particular transistors, displays or organic photovoltaic devices, in particular organic field effect transistors comprising a compound or a polymer in accordance with the present invention.

**Examples**

**Synthesis of XDI-Y-XDI with X being a naphthalene tetracarboxylic acid diimide (NDI) and Y being isoindigo (II)**

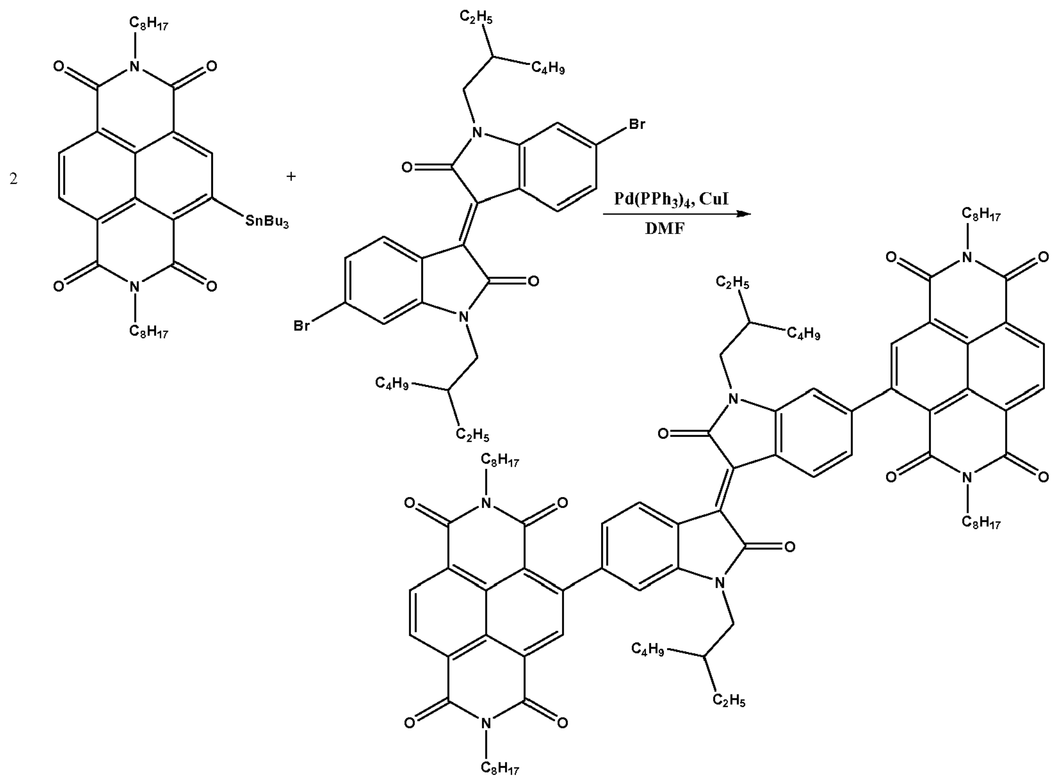


a) ***Synthesis in microwave reactor***

300 mg of N,N'-di-n-octyl-2-(tri-n-butylstannyl)-naphthalene-1,8:4,5-bis(dicarboximide), NDI-$SnBu_3$ (0.385 mmol), 124 mg of isoindigo dibromide (189 mmol, 0.5 eq), 4mg of CuI (0.02 mmol) and 0.02 mmol of $Pd(PPh_3)_4$ in 5 ml of previously degassed anhydrous dimethylformamide were added to a 10 ml vial furnished with the microwave reactor CEM discover. The reaction mixture was magnetically stirred.

The system was allowed to dispense a maximum of 200 W and reached a temperature of 175°C after 90 seconds. The temperature was held for 6 minutes and thereafter the reaction mixture was poured into 75 mL of methanol and filtered and dried. The crude product was analyzed by NMR to containing 66 % by weight of NDI-II-NDI, 12 % by weight of NDI-NDI with yield of 63 % and 12.5 % respectively. The fractions were purified by flash chromatography in dichloromethane with the addition of 1 wt% ethyl acetate.

***b) Synthesis of NDI-II NDI by classical method***

A solution of isoindigo dibromide (0.19 mmol) and *N,N'*-di-*n*-octyl-2-(tri-*n*-butylstannyl)-naphthalene-1,8:4,5-bis(dicarboximide) (NDI-$SnBu_3$, 0.30 g. 0.385 mmol) CuI (4 mg, 0.02 mmol) in dimethylformamide (3 mL) was degassed with argon for 10 minutes. $Pd(PPh_3)_4$ (0.022g, 0.019 mmol) was added and the reaction mixture was heated to 120 °C under nitrogen for 60 minutes. After cooling, the reaction mixture was diluted with methanol (30 mL) and a dark precipitate was collected by filtration. The crude product was purified by column chromatography (silica gel, 1wt% ethyl acetate in dichloromethane). Yield 0.112 g (40%).

The properties of the compounds synthesized in Examples above are summarized in Table 1.

Table 1

| Compound of Example | LUMO (eV) | HOMO (eV) | $\mu$e (mobility) $cm^2/Vs$ |
|---|---|---|---|
| 1 | -3.7 | <-5.8 | 0.1 |

The HOMO and LUMO levels of the organic molecules used in the process of the present invention are determined from cyclic voltammetry measurements in solution as follows:

The measurements are performed at room temperature, under inert atmosphere, with a conventional three-electrode configuration, the solution being outgassed before use with a stream of argon for 5 -10 min. The three-electrode cell may consist e.g of a glassy carbon disk as working electrode, a Pt wire or a Pt rod as a counter electrode and a Pt wire or a carbon rod as pseudo-reference electrode. Ferrocene is used as an internal reference. Other cell configurations may also be used. The solvents used for the determination of the HOMO and LUMO levels are respectively anhydrous

dichloromethane and anhydrous tetrahydrofuran, the supporting electrolyte is 0.1 M tetrabutylammonium hexafluorophosphate and the host concentrations are 2 - 0.5 millimolar. The scan rate is fixed to 100 mv/s.

The HOMO levels ($E_{HOMO}$) of the organic molecules used in the process of the present invention are calculated from the measured half wave potential of their first oxidation wave ($E_1$ ox $_{1/2}$) using the following equation:

$$E_{HOMO} - (-4.8) = -[E_{1\ ox\ 1/2} - E_{ox\ 1/2}(Fc/Fc^+)]$$

wherein the ferrocene HOMO level value has been taken equal to -4.8 eV below the vacuum level according to Pommerehene and al. Adv. Mater. 7(6), 551-554 (1995) and wherein $E_{ox\ 1/2}(Fc/Fc^+)$ corresponds to the measured half wave potential of the ferrocene oxidation wave. For irreversible systems, $Ep_a$ 1 peak potential value of the first oxidation wave is used instead of the half wave potential $E_{10x\ 1/2}$.

The LUMO levels ($E_{LUMO}$) of the organic molecules used in the process of the present invention are calculated from the measured half wave potential of their first reduction wave ($E_{1ox\ 1/2}$) using the following equation:

$$E_{LUMO} - (-4.8) = -[E_{1\ red\ 1/2} - E_{ox\ 1/2}(Fc/Fc^+)]$$

wherein the ferrocene HOMO level value has been taken equal to -4.8 eV below the vacuum level according to Pommerehene et al. Adv. Mater. 7(6), 551-554 (1995) and wherein $E_{ox\ 1/2}(Fc/Fc^+)$ corresponds to the measured half wave potential of the ferrocene oxidation wave. For irreversible systems, $E_{pc\ 1}$ peak potential value of the first reduction wave is used instead of the half wave potential $E_1$ red ½.

If no reduction wave is observed in tetrahydrofuran, the LUMO level is calculated from the HOMO level deduced from cyclic voltammetry as hereabove and from the optical energy bandgap $E_{opt\ g}$ measured from the wavelength of the absorption edge $\lambda_{abs.\ edge}$ using the following equations:

$$E_{opt\ g}\ (eV) = 1240/\lambda_{abs.\ edge}\ (nm);\ E_{LUMO}\ (eV) = E_{HOMO}\ (eV) + E_{opt\ g}\ (eV).$$

If there is a risk of an overlap of the oxidation waves of ferrocene and the compound under evaluation, decamethylferrocene can be introduced into the medium as a reference. The equations used for the calculation of the HOMO and LUMO levels are then:

$$E_{HOMO} - (-4.8 + (E_{ox\ 1/2}(Fc/Fc^+) - E_{ox\ 1/2}(Me_{10}Fc/Me_{10}Fc^+)) = -[E_{1\ ox\ 1/2} - E_{ox\ 1/2}(Me_{10}Fc/Me_{10}Fc^+)]$$

$$E_{LUMO} - (-4.8 + (E_{ox\ 1/2}(Fc/Fc^+) - E_{ox\ 1/2}(Me_{10}Fc/Me_{10}Fc^+)) = -[E_{1\ red\ 1/2} - E_{ox\ 1/2}(Me_{10}Fc/Me_{10}Fc^+)]$$

where $E_{ox\ 1/2}(Fc/Fc^+)$-$E_{ox\ 1/2}(Me_{10}FC/Me_{10}Fc^+)$ are values reported in table 2 of D.Astruc et al., Can.J.Chem.84, 288-299 (2006).

For determining mobility as provided in Table 1, organic thin film transistor devices (OTFT's) with a Top Gate Bottom contact geometry were tested in air using a standard cascade probe station in conjunction with an Agilent B1501 semiconductor parameters analyzer. The Agilent system calculated the saturation mobility according to the equation

$$\mu_{sat} = \frac{2L}{W}\frac{1}{C_i}\left(\frac{\partial I_D^{0.5}}{\partial V_G}\right)^2_{V_D}$$

where L is the transistor length, W is the transistor width, $C_i$ is the dielectric capacitance per unit area, $I_D$ is the drain-source current and $V_G$ is the gate-source voltage. $V_D$ is the drain-source voltage and was set at +100V.

The general structure of the devices used for measurement is shown in Fig. 1 wherein reference numeral 1

designates the Gate (Al), reference numeral 2 designates the dielectric layer, reference numerals 3 and 6 depict self assembled monolayers, reference numeral 4 stands for the drain (Au), reference numeral 5 stands for a buffer layer, reference numeral 7 depicts the source (Au), reference numeral 8 represents the organic semiconductor and reference numeral 9 depicts the bottom (glass).

## Claims

**1.** Compounds of general formulae

XDI-Y-XDI (1)

or

Y'-XDI'-Y' (2)

wherein XDI, which may be the same or different at each occurrence, represents a monovalent rylene tetracarboxylic acid diimide or a monovalent naphthalene tetracarboxylic acid diimide substituent, bound to Y via an aromatic rings wherein one or more of the oxygen atoms of the imide groups may be replaced by sulfur atoms,
XDI', which may be the same or different at each occurrence, represents a divalent rylene tetracarboxylic acid diimide substituent or a divalent naphthalene tetracarboxylic acid diimide substituent, bound to Y' via one or two aromatic rings, wherein one or more of the oxygen atoms of the imide groups may be replaced by sulfur atoms
Y represents a divalent substituent selected from the group consisting of formulae (3) to (6)

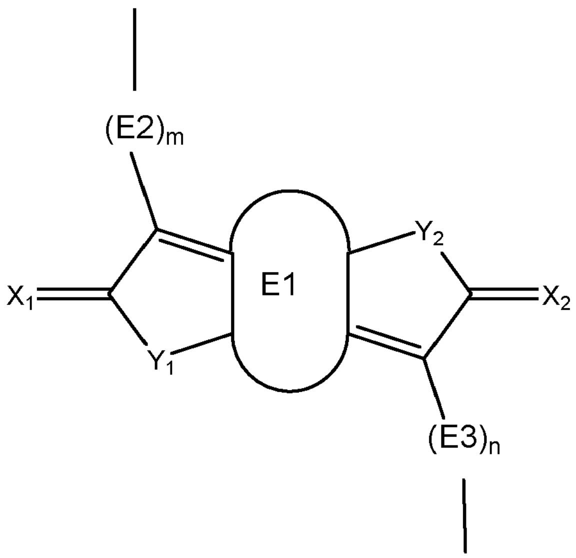

(3)

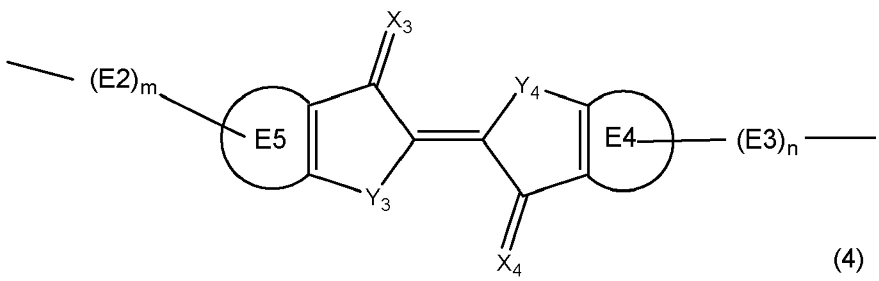

(4)

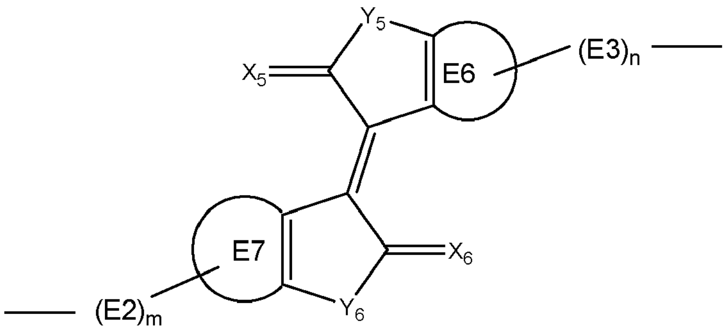

(5)

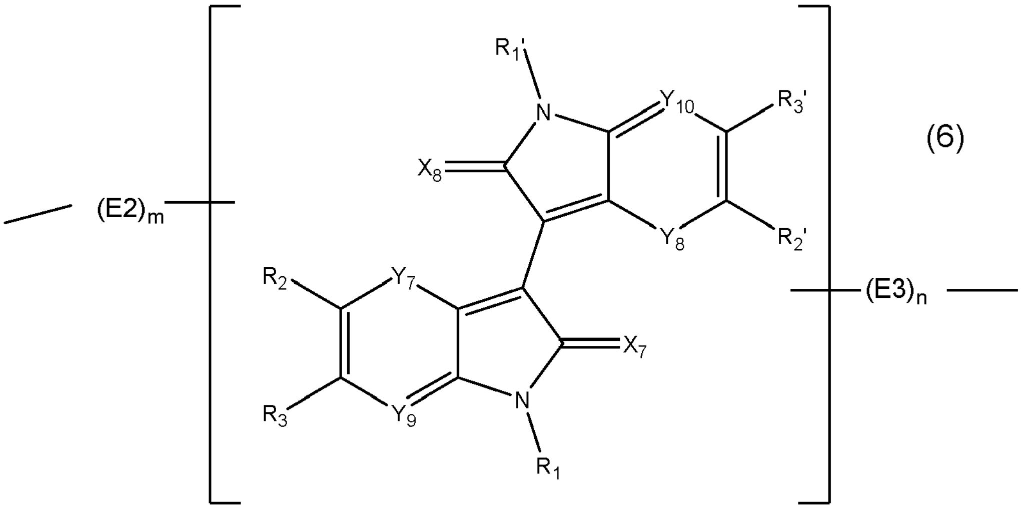

(6)

Y' represents a monovalent substituent represented by formulae (3') to (6')

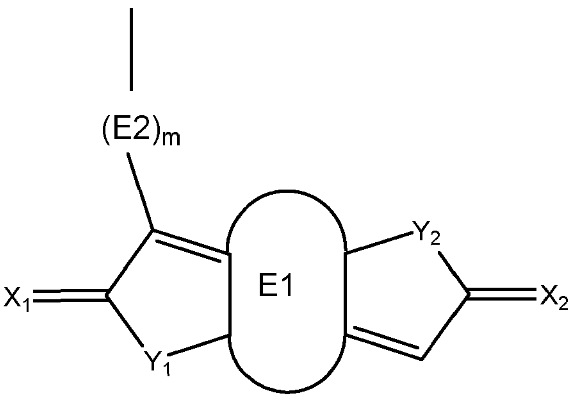

(3')

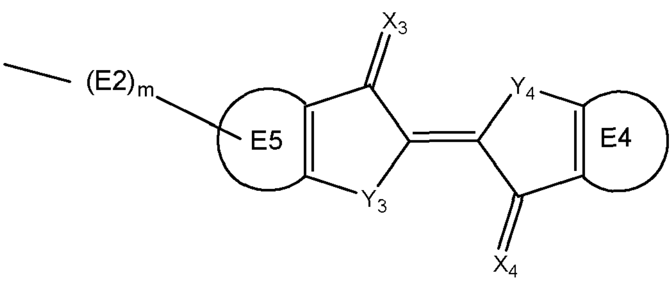

(4')

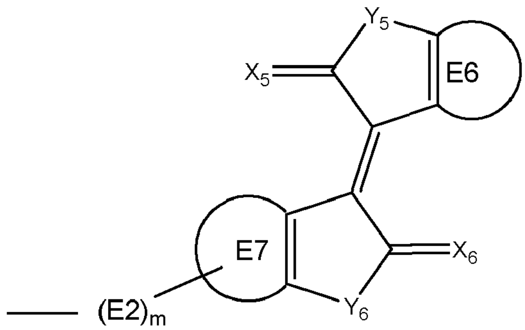

(5')

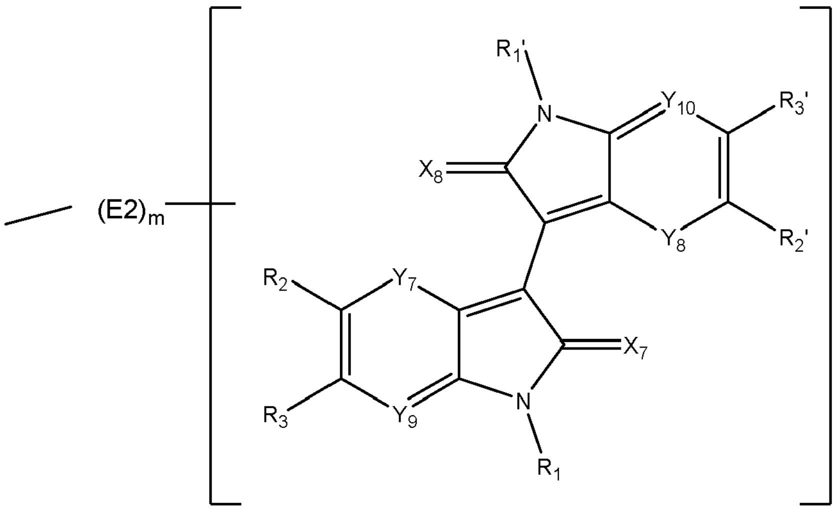

(6')

wherein $X_1$, $X_2$, $X_3$, $X_4$, $X_5$, $X_6$, $X_7$ and $X_8$ which may be the same or different at each occurrence, are selected from O, S or NR,
$Y_1$, $Y_2$, $Y_3$, $Y_4$, $Y_5$, $Y_6$, $Y_7$ and $Y_8$, , which may be the same or different, are selected from O, S or NR,
$Y_9$ and $Y_{10}$, which may be the same or different, are N or P, R, $R_1$ and $R_1$', which may be the same or different, are selected from $C_1$-$C_{20}$ hydrocarbyl or $C_1$-$C_{20}$ heterohydrocarbyl group
$R_2$, $R_2$', $R_3$ and $R_3$', which may be the same or different at each occurrence, represent hydrogen, halogen or a $C_1$-$C_{20}$ hydrocarbyl or $C_1$-$C_{20}$ heterohydrocarbyl group or $R_2$ and $R_3$ respectively $R_2$' and $R_3$' form together a substituted or unsubstituted five or six membered carbocyclic or heterocyclic, aromatic or heteroaromatic ring, which ring may be part of a fused ring system, E1 represents a carbocyclylene, heterocarbocyclylene, arylene or heteroarylene group which is fused through two vicinal carbon atoms with each of the two five membered rings shown in formula (3),
E2 and E3 represent a carbocyclylene, heterocarbocyclylene, arylene or heteroarylene ring system comprising 4 to 14 carbon atoms,
n and m, independent of one another, are 0 or 1,
E4, E5, E6 and E7, which may be the same or different at each occurrence, represent a carbocyclenyl, heterocarbocyclenyl, aryl or heteroaryl ring system comprising 4 to 14 carbon atoms which is fused to the 5- membered ring system shown in formula 4 and 5 through two vicinal carbon atoms.

**2.** Compounds in accordance with claim 1 wherein $X_1$ to $X_6$ represent an oxygen atom.

**3.** Compounds in accordance with claim 1 or claim 2 wherein $Y_1$ to $Y_8$ represent O or NR.

**4.** Compounds of formula (3) in accordance with any of claims 1 to 3 wherein n and m are 1 and E2 and E3 represent an arylene group, preferably phenylene.

**5.** Compounds of formula (4) or (5) in accordance with any of claims 1 to 3 wherein E4 to E7 represent an aryl group, preferably a phenyl group.

6. Compounds of formula (4) or (5) respectively (4') or (5') in accordance with claims 1 to 3 wherein E4 to E7, which may be the same or different at each occurrence, represent a group of formula (7)

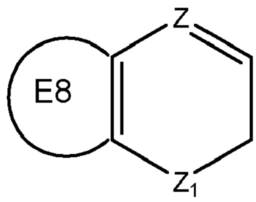

(7)

wherein
E8 represents a substituted or unsubstituted five or six membered aromatic or heteroaromatic ring
Z represents N or P and
$Z_1$ represents O, S, NR" or PR"' where R" and R"' may be the same or different at each occurrence and represent hydrogen or a $C_1$-$C_{20}$ hydrocarbyl or $C_1$-$C_{20}$ heterohydrocarbyl group and wherein the XDI substituent is attached to the aromatic or heteroaromatic ring E8 in formula (7) and the heterocyclyl ring in formula (7) is fused to the five membered ring in formulae (4), (4'), (5) and (5') and wherein both rings may be substituted or unsubstituted.

7. Compounds in accordance with any of claims 1 to 6, wherein XDI, is a bivalent substituent with a core represented by formula (8)

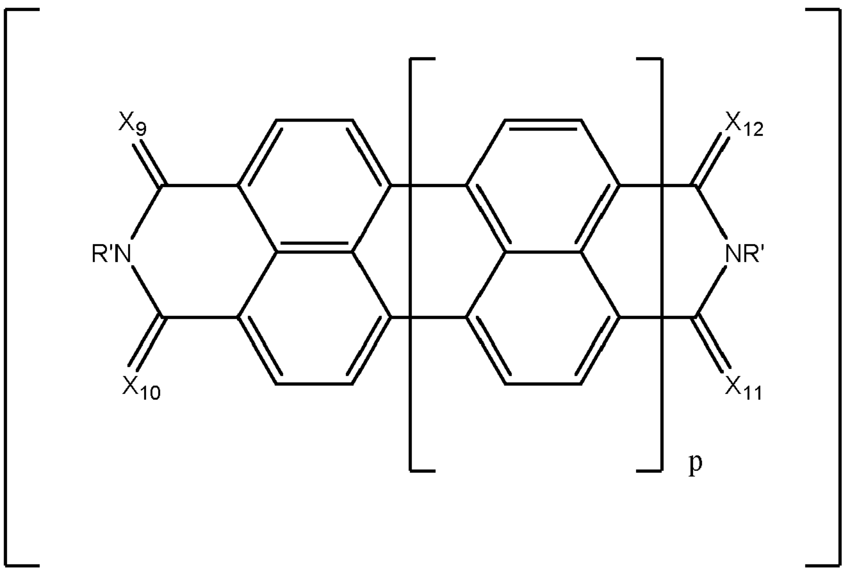

(8)

wherein $X_9$ to $X_{12}$, which may be the same or different, are S or O, R and R' are as defined in claim 1 and p represents an integer of from 0 to 3, preferably 0 or 1. and wherein all aromatic rings may be substituted or unsubstituted and where the bond to Y may be through any of the aromatic rings.

8. Compounds in accordance with any of claims 1 to 7 wherein $Y_9$, $Y_{10}$ and Z are N.

9. Compounds of formula (3) in accordance with any one of claims 1 to 4 or 7 represented by the following formulae

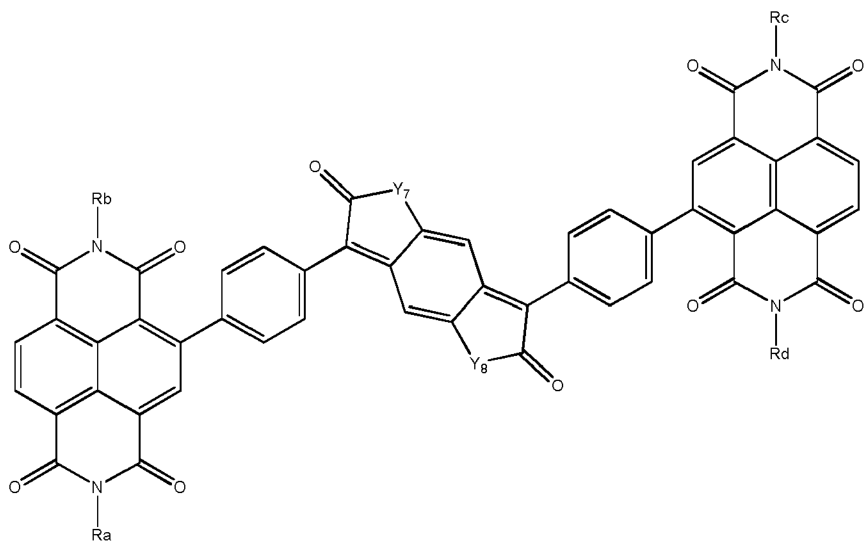

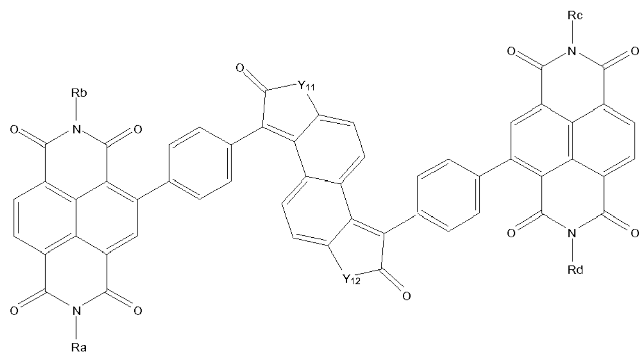

wherein $R_a$ to $R_d$, which may be the same or different, are a $C_1$ to $C_{20}$ linear or branched alkyl group and $Y_{11}$ and $Y_{12}$ represent O, S or $NR_e$ wherein $R_e$ has the meanings as defined for $R_a$ to $R_d$.

**10.** Compounds of formula (4) in accordance with any of claims 1 to 3 and 5 to 7 represented by the following formula

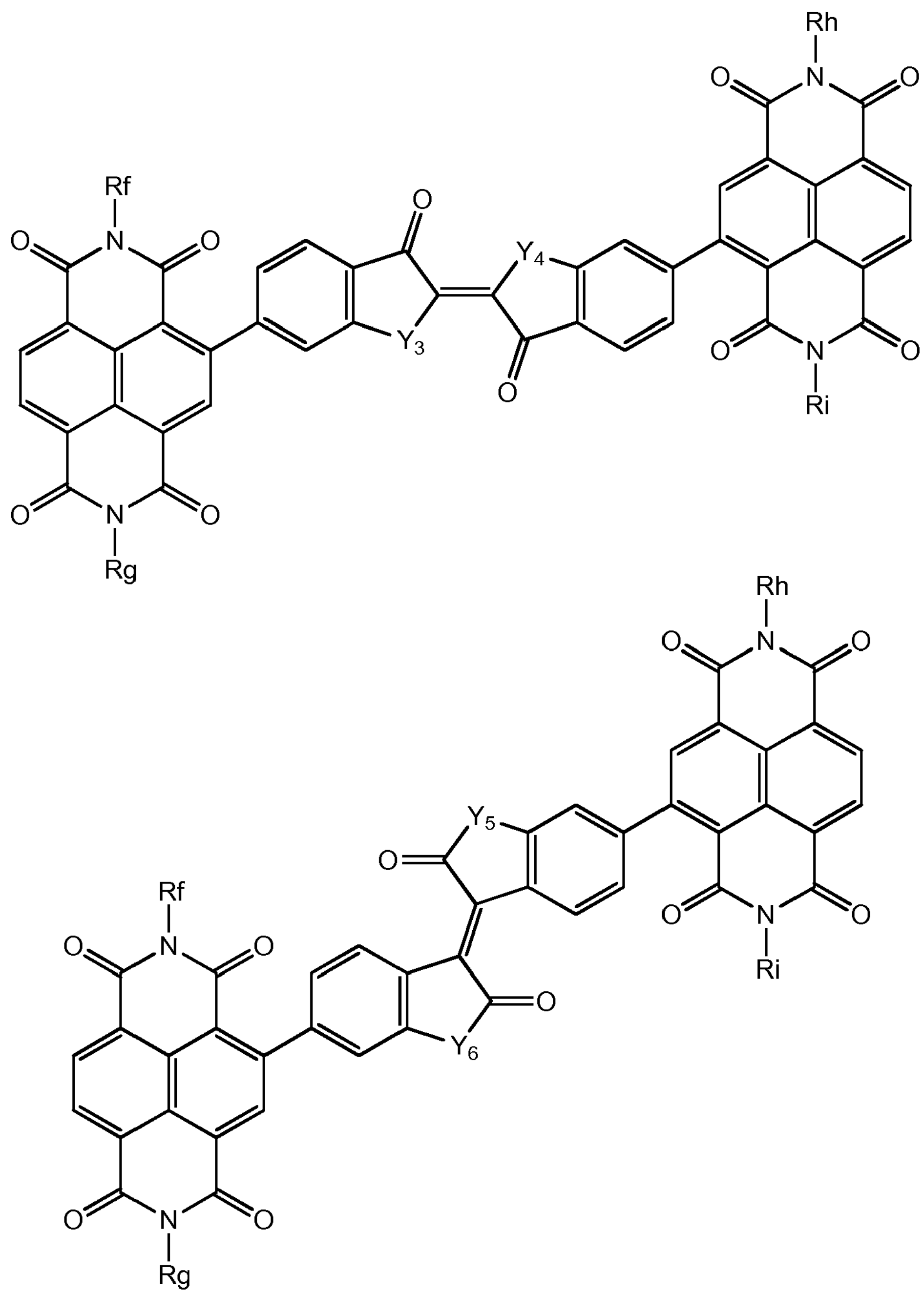

wherein $Y_3$ to $Y_6$, which may be the same or different at each occurrence, are as defined in claim 1 and $R_f$ to $R_i$, which may be the same or different at each occurrence, represent a $C_1$ to $C_{20}$ linear or branched alkyl group.

**11.** Polymers comprising repeating units derived from compounds in accordance with any of claims 1 to 10 containing at least two respective units.

**12.** Use of the compounds in accordance with any of claims 1 to 10 or the polymers in accordance with claim 11 as organic semiconductors in organic electronic devices, in particular as n-type semiconductors in organic field effect transistors.

**13.** Organic electronic devices comprising a compound in accordance with any of claims 1 to 10 or a polymer in accordance with claim 11 or mixtures thereof.

**14.** Organic electronic device in accordance with any of claims 12 and 13 which is a transistor, a display or an organic

photovoltaic device.

15. Organic field effect transistor (OFET) comprising a compound in accordance with any of claims 1 to 10 or a polymer in accordance with claim 11 as organic semiconductor.

Figure 1

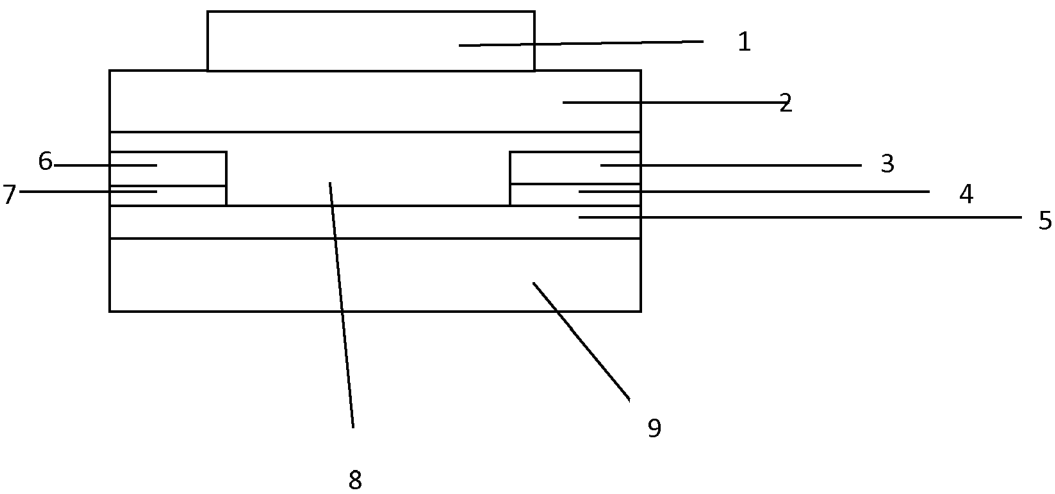

Europäisches Patentamt
European Patent Office
Office européen des brevets

# EUROPEAN SEARCH REPORT

Application Number
EP 13 16 9826

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | XIAOWEI ZHAN ET AL: "Rylene and Related Diimides for Organic Electronics", ADVANCED MATERIALS, vol. 23, no. 2, 11 January 2011 (2011-01-11), pages 268-284, XP055034785, ISSN: 0935-9648, DOI: 10.1002/adma.201001402 * figure 14 * * the whole document * ----- | 1-4,7-9, 11-15 | INV. C09K11/06 H01L51/00 |
| A | WO 2011/025454 A1 (AGENCY SCIENCE TECH & RES [SG]; SONAR PRASHANT [SG]; SINGH SAMARENDRA) 3 March 2011 (2011-03-03) * compound 35 * * compound A5 * * the whole document * ----- | 1-4,7-9, 11-15 | |
| A | MINMIN SHI ET AL: "Design and synthesis of carbonyl group modified conjugated polymers for photovoltaic application", POLYMER BULLETIN, SPRINGER, BERLIN, DE, vol. 68, no. 7, 11 November 2011 (2011-11-11), pages 1867-1877, XP035035857, ISSN: 1436-2449, DOI: 10.1007/S00289-011-0662-1 * page 1870; compounds P(BDT-NDI) * * the whole document * ----- -/-- | 1-4,7-9, 11-15 | TECHNICAL FIELDS SEARCHED (IPC) C09K H01L |

~~The present search report has been drawn up for all claims~~

2

| Place of search | Date of completion of the search | Examiner |
|---|---|---|
| The Hague | 26 February 2014 | Ziegler, Jan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 16 9826

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | QIFAN YAN ET AL: "Conjugated Dimeric and Trimeric Perylenediimide Oligomers", ORGANIC LETTERS, vol. 11, no. 15, 16 July 2009 (2009-07-16), pages 3426-3429, XP055082534, ISSN: 1523-7060, DOI: 10.1021/ol9012734 * scheme 1; compound PpP * * the whole document * | 1-4,7-9, 11-15 | |
| A | LAUREN E. POLANDER ET AL: "Solution-Processed Molecular Bis(Naphthalene Diimide) Derivatives with High Electron Mobility", CHEMISTRY OF MATERIALS, vol. 23, no. 15, 18 July 2011 (2011-07-18), pages 3408-3410, XP055083105, ISSN: 0897-4756, DOI: 10.1021/cm201729s * scheme 1 * * the whole document * | 1-4,7-9, 11-15 | |
| A | CHRISTOF J. KUDLA ET AL: "Cyclopentadithiazole-Based Monomers and Alternating Copolymers", MACROMOLECULES, vol. 43, no. 18, 28 September 2010 (2010-09-28), pages 7864-7867, XP055034813, ISSN: 0024-9297, DOI: 10.1021/ma1014885 * scheme 3 * | 1-4,7-9, 11-15 | TECHNICAL FIELDS SEARCHED (IPC) |

-/--

~~The present search report has been drawn up for all claims~~

2

| Place of search | Date of completion of the search | Examiner |
|---|---|---|
| The Hague | 26 February 2014 | Ziegler, Jan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 16 9826

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | YUFENG WEI ET AL: "Novel Low Bandgap EDOT-Naphthalene Bisimides Conjugated Polymers: Synthesis, Redox, and Optical Properties", MACROMOLECULAR CHEMISTRY AND PHYSICS, vol. 210, no. 9, 6 May 2009 (2009-05-06), pages 769-775, XP055034804, ISSN: 1022-1352, DOI: 10.1002/macp.200900020 * figure 1 * * scheme 2 * * the whole document * ----- | 1-4,7-9, 11-15 | |
| A | DE 10 2011 006885 A1 (LEIBNIZ INST POLYMERFORSCHUNG [DE]) 11 October 2012 (2012-10-11) * figure 1 * * the whole document * ----- | 1-4,7-9, 11-15 | |
| A | CHUNLING GU ET AL: "Naphthalenediimide-Benzothiadiazole Copolymer Semiconductors: Rational Molecular Design for Air-Stable Ambipolar Charge Transport", CHEMISTRY OF MATERIALS, vol. 25, no. 10, 25 April 2013 (2013-04-25), pages 2178-2183, XP055082648, ISSN: 0897-4756, DOI: 10.1021/cm401122h * scheme 1 * * the whole document * ----- | 1-4,7-9, 11-15 | TECHNICAL FIELDS SEARCHED (IPC) |
| | -/-- | | |

~~The present search report has been drawn up for all claims~~

2

| Place of search | Date of completion of the search | Examiner |
|---|---|---|
| The Hague | 26 February 2014 | Ziegler, Jan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 16 9826

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | KAZUHIRO NAKABAYASHI ET AL: "All-Polymer Solar Cells Based on Fully Conjugated Block Copolymers Composed of Poly(3-hexylthiophene) and Poly(naphthalene bisimide) Segments", MACROMOLECULES, vol. 45, no. 24, 10 December 2012 (2012-12-10), pages 9618-9625, XP055082649, ISSN: 0024-9297, DOI: 10.1021/ma302170e * scheme 1 * * the whole document * ----- | 1-4,7-9, 11-15 | |
| A | CN 102 295 754 B (OCEANS KING LIGHTING SCIENCE; OCEANS KING LIGHTING SCIENCE) 26 December 2012 (2012-12-26) * page 5 * * the whole document * ----- | 1-4,7-9, 11-15 | |
| A | WO 2009/098250 A1 (BASF SE [DE]; POLYERA CORP [US]; FACCHETTI ANTONIO [US]; YAN HE [US];) 13 August 2009 (2009-08-13) * paragraphs [0071], [0072] * * claims 1,5 * * the whole document * ----- | 1-4,7-9, 11-15 | TECHNICAL FIELDS SEARCHED (IPC) |
| A | WO 2013/053204 A1 (CHINESE ACAD INST CHEMISTRY [CN]) 18 April 2013 (2013-04-18) * Scheme 3; compounds ZXG-1-16 * * the whole document * ----- | 1-3,5-8, 11-15 | |
| A | WO 2013/053205 A1 (CHINESE ACAD INST CHEMISTRY [CN]) 18 April 2013 (2013-04-18) * claim 1 * * page 10 * * the whole document * ----- -/-- | 1 | |

~~The present search report has been drawn up for all claims~~

2

| Place of search | Date of completion of the search | Examiner |
|---|---|---|
| The Hague | 26 February 2014 | Ziegler, Jan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..............................................................
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 16 9826

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2013/053203 A1 (CHINESE ACAD INST CHEMISTRY [CN]) 18 April 2013 (2013-04-18) * Scheme 4; page 21; compounds ZWY-2-7 * * scheme 3; page 19; compounds ZXG-1-16 * * the whole document * ----- | 1 | |

TECHNICAL FIELDS SEARCHED (IPC)

~~The present search report has been drawn up for all claims~~

2

| Place of search | Date of completion of the search | Examiner |
|---|---|---|
| The Hague | 26 February 2014 | Ziegler, Jan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.....................................................................................................
& : member of the same patent family, corresponding document

EPO FORM 1503 03.82 (P04C01)

Europäisches Patentamt
European Patent Office
Office européen des brevets

Application Number
EP 13 16 9826

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

1-3, 5-8, 11-15

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☒ Only part of the further search fees have been paid within the fixed time limit. The present European search report has been drawn up for those parts of the European patent application which relate to the inventions in respect of which search fees have been paid, namely claims:

1-3, 5-8, 11-15

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches Patentamt
European Patent Office
Office européen des brevets

**LACK OF UNITY OF INVENTION SHEET B**

Application Number
EP 13 16 9826

The Search Division considers that the present European patent application does not comply with the requirements of unity of invention and relates to several inventions or groups of inventions, namely:

1. claims: 4, 9(completely); 1-3, 7, 8, 11-15(partially)

compounds according to formula 1 or formula 2, wherein XDI is selected from monovalent rylene tetracarboxylic acid diimide or
XDI' is selected from divalent rylene tetracarboxylic acid diimide or divalent naphthalene tetracarboxylic acid diimide.
Y is formula 3 or Y' is formula 3'
Polymers, Organic electronic devices and organic field effect transistors comprising such compounds. Use of such compounds as organic semiconductors in organic field effect transistors.

---

2. claims: 10(completely); 1-3, 5-8, 11-15(partially)

compounds according to formula 1 or formula 2, wherein XDI is selected from monovalent rylene tetracarboxylic acid diimide or
XDI' is selected from divalent rylene tetracarboxylic acid diimide or divalent naphthalene tetracarboxylic acid diimide.
Y is formula 4 or Y' is formula 4'
Polymers, Organic electronic devices and organic field effect transistors comprising such compounds. Use of such compounds as organic semiconductors in organic field effect transistors.

---

3. claims: 1-3, 5-8, 11-15(all partially)

compounds according to formula 1 or formula 2, wherein XDI is selected from monovalent rylene tetracarboxylic acid diimide or
XDI' is selected from divalent rylene tetracarboxylic acid diimide or divalent naphthalene tetracarboxylic acid diimide.
Y is formula 5 or Y' is formula 5'
Polymers, Organic electronic devices and organic field effect transistors comprising such compounds. Use of such compounds as organic semiconductors in organic field effect transistors.

---

4. claims: 1, 3, 7, 8, 11-15(all partially)

compounds according to formula 1 or formula 2, wherein XDI is selected from monovalent rylene tetracarboxylic acid diimide or
XDI' is selected from divalent rylene tetracarboxylic acid

Europäisches Patentamt
European Patent Office
Office européen des brevets

**LACK OF UNITY OF INVENTION SHEET B**

Application Number
EP 13 16 9826

The Search Division considers that the present European patent application does not comply with the requirements of unity of invention and relates to several inventions or groups of inventions, namely:

diimide or divalent naphthalene tetracarboxylic acid diimide.
Y is formula 6 or Y' is formula 6'
Polymers, Organic electronic devices and organic field effect transistors comprising such compounds. Use of such compounds as organic semiconductors in organic field effect transistors.

---

## ANNEX TO THE EUROPEAN SEARCH REPORT ON EUROPEAN PATENT APPLICATION NO. EP 13 16 9826

This annex lists the patent family members relating to the patent documents cited in the above-mentioned European search report. The members are as contained in the European Patent Office EDP file on
The European Patent Office is in no way liable for these particulars which are merely given for the purpose of information.

26-02-2014

| Patent document cited in search report | Publication date | Patent family member(s) | Publication date |
|---|---|---|---|
| WO 2011025454 A1 | 03-03-2011 | CN 102549791 A | 04-07-2012 |
| | | CN 102640316 A | 15-08-2012 |
| | | TW 201111410 A | 01-04-2011 |
| | | TW 201111411 A | 01-04-2011 |
| | | US 2012153274 A1 | 21-06-2012 |
| | | US 2012161117 A1 | 28-06-2012 |
| | | WO 2011025454 A1 | 03-03-2011 |
| | | WO 2011025455 A1 | 03-03-2011 |
| DE 102011006885 A1 | 11-10-2012 | DE 102011006885 A1 | 11-10-2012 |
| | | EP 2694567 A2 | 12-02-2014 |
| | | WO 2012136675 A2 | 11-10-2012 |
| CN 102295754 B | 26-12-2012 | NONE | |
| WO 2009098250 A1 | 13-08-2009 | CA 2711764 A1 | 13-08-2009 |
| | | CA 2713083 A1 | 13-08-2009 |
| | | CA 2713852 A1 | 13-08-2009 |
| | | CN 101939351 A | 05-01-2011 |
| | | CN 101939352 A | 05-01-2011 |
| | | CN 101965374 A | 02-02-2011 |
| | | EP 2240528 A1 | 20-10-2010 |
| | | EP 2240529 A1 | 20-10-2010 |
| | | EP 2240530 A1 | 20-10-2010 |
| | | JP 2011514399 A | 06-05-2011 |
| | | JP 2011514913 A | 12-05-2011 |
| | | JP 2011515505 A | 19-05-2011 |
| | | KR 20100115773 A | 28-10-2010 |
| | | KR 20100115786 A | 28-10-2010 |
| | | KR 20100120168 A | 12-11-2010 |
| | | TW 200951159 A | 16-12-2009 |
| | | TW 200951160 A | 16-12-2009 |
| | | TW 201000512 A | 01-01-2010 |
| | | US 2010283047 A1 | 11-11-2010 |
| | | US 2010326527 A1 | 30-12-2010 |
| | | US 2011120558 A1 | 26-05-2011 |
| | | WO 2009098250 A1 | 13-08-2009 |
| | | WO 2009098253 A1 | 13-08-2009 |
| | | WO 2009098254 A1 | 13-08-2009 |
| WO 2013053204 A1 | 18-04-2013 | CN 103044661 A | 17-04-2013 |
| | | WO 2013053204 A1 | 18-04-2013 |
| WO 2013053205 A1 | 18-04-2013 | CN 103044662 A | 17-04-2013 |
| | | WO 2013053205 A1 | 18-04-2013 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 13 16 9826

This annex lists the patent family members relating to the patent documents cited in the above-mentioned European search report. The members are as contained in the European Patent Office EDP file on
The European Patent Office is in no way liable for these particulars which are merely given for the purpose of information.

26-02-2014

| Patent document cited in search report | Publication date | Patent family member(s) | Publication date |
|---|---|---|---|
| WO 2013053203 A1 | 18-04-2013 | CN 103044660 A | 17-04-2013 |
| | | WO 2013053203 A1 | 18-04-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012142460 A


### Non-patent literature cited in the description


- **MARKS et al.** *Adv. Mater.,* 2011, vol. 23, 268-284
- **YAN ; ZHAO.** *Org. Lett.,* 2009, vol. 11 (15), 3426-3419
- **POMMEREHENE.** *Adv. Mater.,* 1995, vol. 7 (6), 551-554
- **POMMEREHENE et al.** *Adv. Mater.,* 1995, vol. 7 (6), 551-554
- **D.ASTRUC et al.** *Can.J.Chem.,* 2006, vol. 84, 288-299